(19) Europäisches Patentamt
European Patent Office
Office européen des brevets

(11) **EP 2 324 069 B1**

(12) **EUROPÄISCHE PATENTSCHRIFT**

(45) Veröffentlichungstag und Bekanntmachung des Hinweises auf die Patenterteilung:
**18.12.2013 Patentblatt 2013/51**

(21) Anmeldenummer: **09782368.6**

(22) Anmeldetag: **31.08.2009**

(51) Int Cl.:
***C08F 220/18*** (2006.01) ***C08F 220/26*** (2006.01)
***C08F 220/28*** (2006.01) ***C09D 133/00*** (2006.01)
***C09D 133/14*** (2006.01)

(86) Internationale Anmeldenummer:
**PCT/EP2009/061177**

(87) Internationale Veröffentlichungsnummer:
**WO 2010/026118 (11.03.2010 Gazette 2010/10)**

(54) **MONOMERMISCHUNG, POLYMER, BESCHICHTUNGSMITTEL UND VERFAHREN ZUR HERSTELLUNG EINER BESCHICHTUNG**

MONOMER MIXTURE, POLYMER, COATING MEANS AND METHOD FOR PRODUCING A COATING

MÉLANGE DE MONOMÈRES, POLYMÈRE, AGENT DE REVÊTEMENT ET PROCÉDÉ DE PRODUCTION D'UN REVÊTEMENT

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **08.09.2008 DE 102008046076**
**08.06.2009 DE 102009026819**

(43) Veröffentlichungstag der Anmeldung:
**25.05.2011 Patentblatt 2011/21**

(73) Patentinhaber: **Evonik Röhm GmbH**
**64293 Darmstadt (DE)**

(72) Erfinder:
- **BREINER, Christine, Maria**
**69514 Laudenbach (DE)**
- **SCHMITT, Gerold**
**63743 Aschaffenburg (DE)**
- **KLESSE, Wolfgang**
**55127 Mainz (DE)**
- **GOMEZ ANDREU, Mario**
**64319 Pfungstadt (DE)**

(56) Entgegenhaltungen:
**EP-A2- 0 005 167 DE-A1- 1 817 716**
**DE-A1- 2 900 843 US-A- 4 191 838**

- **ZABRANSKY, HOUSKA, PLITCHA, KALAL: "Polymers with aldehyde and acetal groups based om esters of methacrylic acid" MAKROMOLEKULARE CHEMIE, Bd. 186, 1985, Seiten 231-245, XP002564767**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 324 069 B1

## Beschreibung

Die vorliegende Erfindung betrifft eine Monomermischung und ein Polymer, das unter Verwendung dieser Monomermischung erhältlich ist. Darüber hinaus richtet sich die vorliegende Erfindung auf ein Beschichtungsmittel, ein Verfahren zur Herstellung einer Beschichtung und einen beschichteten Gegenstand.

Beschichtungsmittel, insbesondere Lacke werden seit langer Zeit synthetisch hergestellt. Neuere Beschichtungsmittel umfassen Carbonylgruppen-haltige Polymere, die durch Zugabe von Vernetzungsmitteln zu relativ lösungsmittelbeständigen Lacken gehärtet werden können. Diese Beschichtungsmittel werden unter anderem in WO 94/025433 dargelegt. Allerdings ist die Verbesserung des Eigenschaftsprofils dieser Beschichtungsmittel ein dauerhaftes Bedürfnis.

DE 2900843 offenbart Polymerisationsprodukte zum Beschichten von Leder, Textil oder Papier.

EP0005167 beschreibt Bindemittel für Anstricht mittel auf der Basis von wässrigen Dispersion enthaltend Carbonylgruppen aufweisenden Monomeren.

US4191838 beschreibt die Verwendung von Alkyl(Meth)acrylate Monomere enthaltend Aldehydgruppe im Alkylrest für die Herstellung von Polymere.

In Anbetracht des Standes der Technik ist es nun Aufgabe der vorliegenden Erfindung Monomermischungen zur Verfügung zu stellen, die sich zu Polymeren mit hervorragenden Eigenschaften verarbeiten lassen. Zu diesen Eigenschaften gehören insbesondere Merkmale, die durch Beschichtungsmittel und Beschichtungen, die aus den Beschichtungsmitteln erhältlich sind, offenbar werden.

Insbesondere sollten sich die Monomermischungen zu Dispersionen bzw. zu Polymeren, beispielsweise Emulsionspolymeren, verarbeiten lassen, die einen sehr geringen Restmonomergehalt aufweisen.

Weiterhin war es mithin eine Aufgabe der vorliegenden Erfindung ein Beschichtungsmittel bereitzustellen, das eine besonders lange Lagerfähigkeit und Haltbarkeit aufweist. Des Weiteren sollte die Härte der Beschichtungen, die aus den Beschichtungsmitteln erhältlich sind, über einen weiten Bereich variiert werden können. Insbesondere sollten mechanisch stabile Beschichtungen erhalten werden können, die sich durch eine hohe Bruchdehnung und/oder eine hohe Zugfestigkeit auszeichnen. Weiterhin sollten die aus den Beschichtungsmitteln erhältlichen Beschichtungen eine geringe Sprödigkeit, bezogen auf die Härte und die Zugfestigkeit, zeigen.

Eine weitere Aufgabe ist darin zu sehen Polymere zur Verfügung zu stellen, durch deren Verwendung Beschichtungsmittel ohne flüchtige organische Lösungsmittel erhältlich sind. Die aus den Beschichtungsmitteln erhältlichen Beschichtungen sollten eine hohe Witterungsbeständigkeit, insbesondere eine hohe UV-Beständigkeit aufweisen. Darüber hinaus sollten die aus den Beschichtungsmitteln erhältlichen Filme nach kurzer Zeit eine geringe Klebrigkeit aufweisen.

Weiterhin sollten die aus den Polymeren bzw. Monomermischungen erhältlichen Beschichtungen eine besondere hohe Beständigkeit gegenüber Lösungsmitteln aufweisen. Hierbei sollte diese Stabilität gegenüber vielen unterschiedlichen Lösungsmitteln hoch sein. Ebenso sollte eine sehr gute Beständigkeit gegenüber sauren und alkalischen Reinigungsmitteln gegeben sein.

Ferner war es mithin Aufgabe der vorliegenden Erfindung Monomermischungen, Polymere und Beschichtungsmittel anzugeben, die besonders kostengünstig erhältlich sind. Hierbei sollten diese Produkte und Verfahren zur Herstellung derselben umweltverträglich sein. Hinsichtlich der Polymere ist auszuführen, dass diese, bei gleicher Leistungsfähigkeit, einen möglichst geringen Anteil an aufwendig herzustellenden Monomeren aufweisen sollten.

Eine weitere Aufgabe kann darin gesehen werden, ein Beschichtungsmittel für Textilien und Leder bereitzustellen, die für diesen Einsatzzweck besonders gut geeignet sind. Insbesondere sollten die Beschichtungen eine hohe Abriebfestigkeit und eine ausreichend hohe Elastizität bei ausgezeichneter Festigkeit zeigen. Insbesondere sollten die Beschichtungen eine ausgezeichnete Chemikalienbeständigkeit, insbesondere gegen Reinigungsmitteln; und einen geringen Kochwaschverlust aufweisen.

Darüber hinaus war es eine Aufgabe der vorliegenden Erfindung, Beschichtungsmittel für Bodenbeläge, Möbel und andere relativ feste Substrate bereitzustellen, die eine ausgezeichnete mechanische Belastbarkeit bei hoher Chemikalienresistenz zeigen.

Beispielsweise sollten Bindemittel für Industrielacke und Grundierungen bereitgestellt werden, die ein hervorragendes Eigenschaftsprofil aufweisen. So sollten die Lacke im Innen- oder Außenbereich von Gebäuden, zum Beispiel als Fassadenfarbe, eingesetzt werden können.

Ferner sollten die Lacke auf Holz, Papier, Metall und Kunststoff angewendet werden können, wobei insbesondere Klarlacke für Holz, Industrielack für Holz, Industrie-Grundierung für Holz zu nennen sind. Hierbei sollten die Beschichtungsmittel eine ausgezeichnete Haftung, Flexibilität und Korrosionsbeständigkeit zeigen.

Gelöst werden diese sowie weitere nicht explizit genannte Aufgaben, die jedoch aus den hierin einleitend diskutierten Zusammenhängen ohne weiteres ableitbar oder erschließbar sind, durch eine Monomermischung mit allen Merkmalen des Patentanspruchs 1. Zweckmäßige Abwandlungen der erfindungsgemäßen Monomermischungen werden in Unteransprüchen unter Schutz gestellt. Hinsichtlich eines Polymers, eines Beschichtungsmittels, eines Verfahrens zur Herstellung einer Beschichtung sowie eines beschichteten Gegenstandes liefern die Ansprüche 16, 20, 21 und 25

eine Lösung der zugrunde liegenden Aufgaben.

Gegenstand der vorliegenden Erfindung ist dementsprechend eine Monomermischung umfassend mindestens 30 Gew.-% eines oder mehrerer Alkyl(meth)acrylate mit 1 bis 10 Kohlenstoffatomen im Alkylrest, deren Alkylrest keine Doppelbindungen oder Heteroatome aufweist, und
0,1 bis 40 Gew.-% eines oder mehrerer (Meth)acrylate mit mindestens einer Aldehydgruppe im Alkylrest, dadurch gekennzeichnet, dass das (Meth)acrylat mit mindestens einer Aldehydgruppe im Alkylrest am Kohlenstoffatom in alpha-Stellung zur Aldehydgruppe ein Wasserstoffatom aufweist.

Durch die erfindungsgemäßen Maßnahmen können des Weiteren unter anderem die folgenden Vorteile erzielt werden:

Die erfindungsgemäßen Monomermischungen lassen sich zu Polymeren, Beschichtungsmitteln und Beschichtungen verarbeiten, die einen sehr geringen Restmonomergehalt aufweisen.

Die Härte der Beschichtungen, die aus erfindungsgemäßen Beschichtungsmitteln erhältlich sind, die wiederum auf den Polymeren bzw. Monomermischungen basieren, kann über einen weiten Bereich variiert werden. Gemäß einer bevorzugten Abwandlung können insbesondere mechanisch stabile Beschichtungen erhalten werden. Die aus den Beschichtungsmitteln der vorliegenden Erfindung erhältlichen Beschichtungen zeigen eine überraschend hohe Lösungsmittelbeständigkeit, die sich insbesondere in Versuchen mit Methylisobutylketon (MIBK) oder Ethanol zeigt.

Überraschend können insbesondere mechanisch stabile Beschichtungen erhalten werden, die sich durch eine hohe Bruchdehnung und/oder eine hohe Zugfestigkeit auszeichnen. Vorzugsweise zeigen die aus den Beschichtungsmitteln erhältlichen Beschichtungen eine geringe Sprödigkeit, bezogen auf die Härte und die Zugfestigkeit.

Beschichtungsmittel, die unter Verwendung der erfindungsgemäßen Monomermischungen erhältlich sind, benötigen im Allgemeinen keine flüchtigen organischen Lösungsmittel. Darüber hinaus zeigen die erfindungsgemäßen Beschichtungsmittel eine hohe Lagerungsbeständigkeit, eine hohe Haltbarkeit und eine sehr gute Lagerfähigkeit. Insbesondere tritt kaum eine Aggregatbildung auf.

Die aus den erfindungsgemäßen Beschichtungsmitteln erhältlichen Beschichtungen zeigen eine hohe Witterungsbeständigkeit, insbesondere eine hohe UV-Beständigkeit. Des Weiteren weisen die aus den Beschichtungsmitteln erhältlichen Filme nach kurzer Zeit eine geringe Klebrigkeit auf.

Die erfindungsgemäßen Monomermischungen, Polymere und Beschichtungsmittel lassen sich kostengünstig in großem Maßstab herstellen. Hinsichtlich der Polymere ist auszuführen, dass diese, bei gleicher Leistungsfähigkeit, einen geringeren Anteil an aufwendig herzustellenden Monomeren aufweisen können. Die Leistungsfähigkeit der Polymere ergibt sich unter anderem aus den Eigenschaften der hieraus erhältlichen Beschichtungsmitteln und Beschichtungen.

Die erfindungsgemäßen Beschichtungsmittel sind umweltfreundlich und können sicher und ohne großen Aufwand verarbeitet und hergestellt werden. Hierbei zeigen die erfindungsgemäßen Beschichtungsmittel eine sehr hohe Scherstabilität.

Weiterhin können die vorliegenden Beschichtungsmittel insbesondere zur Beschichtung von Textilien und Leder eingesetzt werden. Insbesondere zeigen die hieraus erhältlichen Beschichtungen eine hohe Abriebfestigkeit und eine ausreichend hohe Elastizität bei ausgezeichneter Festigkeit.

Darüber hinaus können besondere Ausgestaltungen eines erfindungsgemäßen Beschichtungsmittels auf harten Substraten, wie zum Beispiel Holz, Metall und Kunststoffen eingesetzt werden, wobei die hierdurch erhaltenen Beschichtungen ausgezeichnete Eigenschaften aufweisen. So zeigen die erhaltenen Beschichtungen insbesondere bei Versuchen gemäß dem Möbeltest DIN 68861-1 eine hervorragende Einstufung. Weiterhin sind die mechanischen Eigenschaften ausgezeichnet. Insbesondere weisen bevorzugte Beschichtungen eine hohe Härte auf, die bei einer geringen Sprödigkeit der Beschichtung erzielt werden kann. Darüber hinaus können die Beschichtungen eine hohe Zugfestigkeit und eine hohe Bruchdehnung zeigen.

Weiterhin können erfindungsgemäße Bindemittel für Industrielacke und Grundierungen eingesetzt werden, da diese ein hervorragendes Eigenschaftsprofil aufweisen. Beispielsweise können die Lacke im Innen- oder Außenbereich von Gebäuden, zum Beispiel als Fassadenfarbe, eingesetzt werden.

Ferner können die Lacke auf Holz, Papier, Metall und Kunststoff angewendet werden, wobei sich diese insbesondere als Klarlacke für Holz, Industrielack für Holz, Industrie-Grundierung für Holz eignen. Hierbei können die Beschichtungsmittel eine ausgezeichnete Haftung, Flexibilität und Korrosionsbeständigkeit zeigen.

Die erfindungsgemäße Monomermischung weist 0,1 bis 40 Gew.-%, vorzugsweise 1 bis 20 Gew.-%, besonders bevorzugt 1,5 bis 10 Gew.-% und ganz besonders bevorzugt 2 bis 6 Gew.-% eines oder mehrerer (Meth)acrylate mit mindestens einer Aldehydgruppe im Alkylrest auf.

(Meth)acrylate mit mindestens einer Aldehydgruppe im Alkylrest entsprechen im Allgemeinen der allgemeinen Formel (I)

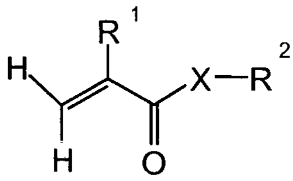

(I),

worin $R^1$ Wasserstoff oder eine Methylgruppe, X Sauerstoff oder eine Gruppe der Formel NR', worin R' Wasserstoff oder ein Rest mit 1 bis 6 Kohlenstoffatomen ist, $R^2$ ein Rest mit 3 bis 31 Kohlenstoffatomen und mindestens einer Aldehydgruppe ist.

Der Ausdruck "Rest mit 1 bis 6 Kohlenstoffatomen" bzw. "Rest mit 3 bis 31 Kohlenstoffatomen" steht für eine Gruppe, die 1 bis 6 bzw. 3 bis 31 Kohlenstoffatome aufweist. Er umfasst aromatische und heteroaromatische Gruppen sowie Alkyl-, Cycloalkyl-, Alkoxy-, Cycloalkoxy-, Alkenyl-, Alkanoyl-, Alkoxycarbonylgruppen sowie heteroalipatische Gruppen. Dabei können die genannten Gruppen verzweigt oder nicht verzweigt sein. Des Weiteren können diese Gruppen Substituenten, insbesondere Halogenatome oder Hydroxygruppen aufweisen.

Vorzugsweise stehen die Reste R', für Alkylgruppen. Zu den bevorzugten Alkylgruppen gehören die Methyl-, Ethyl-, Propyl-, Isopropyl-, 1-Butyl-, 2-Butyl-, 2-Methylpropyl-, tert.-Butyl-Gruppe.

In Formel (I) bedeutet der Rest $R^2$ eine Gruppe mit 3 bis 31 Kohlenstoffatomen, insbesondere mit 3 bis 25, vorzugsweise mit 3 bis 9, besonders bevorzugt mit 4 bis 6 Kohlenstoffatomen, der mindestens eine Aldehydgruppe umfasst. Gemäß einer weiteren Ausgestaltung der vorliegenden Erfindung sind (Meth)acrylatmonomere bevorzugt, die 10 bis 25, vorzugsweise 12 bis 24 und ganz besonders bevorzugt 14 bis 23 Kohlenstoffatome aufweisen. Hierbei kann der Rest $R^2$ ein, zwei, drei oder mehr Aldehydgruppen umfassen, wobei der Rest $R^2$ substituiert sein kann und weitere funktionale Gruppen, beispielsweise C-C-Doppelbindungen enthalten kann. Gemäß einer bevorzugten Abwandlung der vorliegenden Erfindung stellt der Rest $R^2$ eine Alkyl- oder Alkenylgruppe dar, die eine oder zwei Aldehydgruppen umfasst, wobei Reste mit genau einer

Aldehydgruppe besonders bevorzugt sind. Diese Gruppe kann Heteroatome, insbesondere Sauerstoff- und/oder Stickstoffatome, beispielsweise als Ester-, Ether-, Amino- und/oder Amidgruppe umfassen.

Gemäß einer bevorzugten Ausführungsform kann das (Meth)acrylat mit mindestens einer Aldehydgruppe ein Enol bzw. Enolat bilden. Dementsprechend weisen bevorzugte (Meth)acrylate mit mindestens einer Aldehydgruppe am Kohlenstoffatom in alpha-Stellung zur Aldehydgruppe mindestens ein Wasserstoffatom auf.

Zu den bevorzugten Resten $R^2$ zählt insbesondere die 1-Formylethyl-, 2-Formylethyl-, 2-Formylpropyl-, 3-Formylpropyl-, 2-Formyl-octa-7-enyl-, 2,7-Diformyloctyl-, 9-Formyloctadecyl- und 10- Formyloctadecyl-Gruppe.

Zu den bevorzugten (Meth)acrylatmonomeren gemäß Formel (I) gehören unter anderem (Meth)acrylatmonomere mit 3 bis 9 Kohlenstoffatomen im Rest $R^2$, wie zum Beispiel 3-Oxopropyl(meth)acrylat (2-Formylethyl(meth)acrylat), 4-Oxobutyl-(meth)acrylat (3-Formylpropyl(meth)acrylat) und 2-Methyl-3-oxopropyl(meth)acrylat (2-Formyl-2-methylethyl(meth)acrylat), wobei 4-Oxobutylmethacrylat und 2-Methyl-3-oxopropylmethacrylat besonders bevorzugt sind.

Darüber hinaus gehören zu den (Meth)acrylatmonomeren gemäß Formel (I) (Meth)acrylatmonomere mit 10 bis 25 Kohlenstoffatomen im Rest $R^2$, wie (Meth)acrylate, die sich von Fettsäuren, Fettalkoholen und Fettsäureamiden ableiten, wie

9-Formyloctadecan-12-en-yl-(meth)acrylat, 9,12-Diformyloctadecyl-(meth)acrylat, 12-Formyloctadecan-6,9-dienyl-(meth)acrylat, 9-Formylhexadecyl(meth)acrylat, 10-Formylhexadecyl(meth)acrylat, 9-Formyloctadecyl(meth)acrylat, 10-Formyloctadecyl(meth)acrylat, (Meth)acryloyloxy-2-hydroxypropyl-9-formyloctadecansäureester, (Meth)acryloyloxy-2-hydroxypropyl-10-formyloctadecansäureester, (Meth)acryloyloxy-2-hydroxypropyl-9-formyloctadecansäureamid und/oder (Meth)acryloyloxy-2-hydroxypropyl-10-formyloctadecansäureamid.

Die genannten Monomere können einzeln oder als Mischung von zwei oder mehr Verbindungen eingesetzt werden.

Vorteile, die an sich für den Fachmann nicht nahe liegen, können durch eine Monomermischung erzielt werden, die 1 bis 8 Gew.-%, besonders bevorzugt 2 bis 6 Gew.-% (Meth)acrylate mit mindestens einer Aldehydgruppe im Alkylrest aufweist, bezogen auf das Gesamtgewicht der Monomere.

Neben mindestens einem (Meth)acrylat mit mindestens einer Aldehydgruppe im Alkylrest umfasst eine erfindungsgemäße Monomermischung mindestens 30 Gew.-%, vorzugsweise mindestens 55 Gew.-% und ganz bevorzugt mindestens 70 Gew.-% eines oder mehrerer Alkyl(meth)acrylate mit 1 bis 10 Kohlenstoffatomen im Alkylrest, deren Alkylrest keine Doppelbindungen oder Heteroatome aufweist. Der Ausdruck (Meth)acrylate umfasst Methacrylate und Acrylate sowie Mischungen derselben. Diese Monomere sind weithin bekannt.

Hierzu gehören insbesondere Alkyl(meth)acrylate, die sich von linearen oder verzweigten gesättigten Alkoholen ableiten, wie zum Beispiel Methyl(meth)acrylat, Ethyl(meth)acrylat, n-Propyl(meth)acrylat, iso-Propyl(meth)acrylat, n-Butyl(meth)acrylat, iso-Butyl(meth)acrylat, tert.-Butyl(meth)acrylat, Pentyl(meth)acrylat, Hexyl(meth)acrylat, 2-Ethylhexyl(meth)acrylat, Heptyl(meth)acrylat, Octyl(meth)acrylat,

3-iso-Propylheptyl(meth)acrylat, Nonyl(meth)acrylat, Decyl(meth)acrylat; und Cycloalkyl(meth)acrylate, wie Cyclopentyl (meth)acrylat, Cyclohexyl(meth)acrylat, Cyclohexyl(meth)acrylate mit mindestens einem Substituenten am Ring, wie tert-Butylcyclohexyl(meth)acrylat und Trimethylcyclohexyl(meth)acrylat, Norbornyl(meth)acrylat, Methylnorbornyl(meth) acrylat, Dimethylnorbornyl(meth)acrylat, Bornyl(meth)acrylat, 1-Adamantyl(meth)acrylat, 2-Adamantyl(meth)acrylat, Menthyl(meth)acrylat und Isobornyl(meth)acrylat.

Gemäß einer besonderen Ausführungsform sind insbesondere Monomermischungen von Interesse, die Alkylacrylate mit 1 bis 10 Kohlenstoffatomen im Alkylrest umfassen, deren Alkylrest keine Doppelbindungen oder Heteroatome aufweist.

Hierzu gehören insbesondere Alkylacrylate, die sich von gesättigten Alkoholen ableiten, wie zum Beispiel Methylacrylat, Ethylacrylat, n-Propylacrylat, iso-Propylacrylat, n-Butylacrylat, tert.-Butylacrylat, Pentylacrylat, Hexylacrylat, Heptylacrylat, Octylacrylat und Ethylhexylacrylat; und Cycloalkylacrylate, wie Cyclopentylacrylat und Cyclohexylacrylat. Von den genannten Alkylacrylaten sind Ethylacrylat, Butylacrylat und Ethylhexylacrylat besonders bevorzugt, wobei Butylacrylat ganz besonders bevorzugt ist.

Besonders bevorzugte Monomermischungen gemäß dem ersten Aspekt der vorliegenden Erfindung können mindestens 30 Gew.-%, vorzugsweise mindestens 55 Gew.-% und ganz bevorzugt mindestens 70 Gew.-% eines oder mehrerer Alkylacrylate mit 1 bis 10 Kohlenstoffatomen im Alkylrest enthalten, deren Alkylrest keine Doppelbindungen oder Heteroatome aufweist.

Gemäß einer besonderen Ausführungsform der vorliegenden Erfindung sind insbesondere Monomermischungen bevorzugt, die Methacrylate mit 1 bis 10 Kohlenstoffatomen im Alkylrest enthalten, deren Alkylrest keine Doppelbindungen oder Heteroatome aufweist.

Hierzu gehören insbesondere Alkylmethacrylate mit 1 bis 10 Kohlenstoffatomen im Alkylrest, die sich von gesättigten Alkoholen ableiten, wie Methylmethacrylat, Ethylmethacrylat, n-Propylmethacrylat, iso-Propylmethacrylat, n-Butylmethacrylat, tert.-Butylmethacrylat und Pentylmethacrylat, Hexylmethacrylat,
2-Ethylhexylmethacrylat, Heptylmethacrylat, Octylmethacrylat, 3-iso-Propylheptylmethacrylat, Nonylmethacrylat, Decylmethacrylat, und Cycloalkylmethacrylate, wie Cyclopentylmethacrylat, Cyclohexylmethacrylat und Isobornylmethacrylat. Hierbei sind Methylmethacrylat und Cycloalkylmethacrylate besonders bevorzugt.

Vorzugsweise kann eine Monomermischung gemäß der ersten Ausführungsform 0 bis 60 Gew.-%, bevorzugt 10 bis 50 Gew.-% und ganz besonders bevorzugt 20 bis 40 Gew.-% Alkylmethacrylate mit 1 bis 10 Kohlenstoffatomen im Alkylrest, deren Alkylrest keine Doppelbindungen oder Heteroatome aufweist, umfassen.

Überraschende Vorteile können insbesondere durch Monomermischungen gemäß der ersten Ausführungsform erzielt werden, die sich durch ein relativ hohes Gewichtsverhältnis von Alkylacrylaten mit 1 bis 10 Kohlenstoffatomen im Alkylrest zu Alkylmethacrylaten mit 1 bis 10 Kohlenstoffatomen im Alkylrest auszeichnen. Vorzugsweise kann das Gewichtsverhältnis von Alkylacrylat mit 1 bis 10 Kohlenstoffatomen im Alkylrest zu Alkylmethacrylat mit 1 bis 10 Kohlenstoffatomen im Alkylrest im Bereich von 1:1 bis 50:1, besonders bevorzugt 3:2 bis 5:1 liegen.

Monomermischungen gemäß der ersten Ausführungsform eignen sich insbesondere zur Herstellung von Polymeren bzw. Beschichtungsmitteln, die auf sehr flexible Materialien, insbesondere Geweben, Gewirken und Vliesen Anwendung finden. Dementsprechend können diese Beschichtungsmittel insbesondere auf Textilien, Leder und/oder Vlies, wie Faservliese oder Vliesstoffe, eingesetzt werden. Hierbei unterliegen die zu beschichtenden Materialien keiner besonderen Beschränkung, so dass die Beschichtungsmittel auf Natur-, Kunststoff- und/oder Glasfasern, insbesondere Glasfaservliesen eingesetzt werden können.

Besonders bevorzugte Monomermischungen gemäß einem zweiten Aspekt der vorliegenden Erfindung können mindestens 30 Gew.-%, vorzugsweise mindestens 55 Gew.-% und ganz bevorzugt mindestens 70 Gew.-% eines oder mehrerer Alkylmethacrylate mit 1 bis 10 Kohlenstoffatomen im Alkylrest enthalten, deren Alkylrest keine Doppelbindungen oder Heteroatome aufweist.

Vorzugsweise kann eine Monomermischung gemäß der zweiten Ausführungsform 0 bis 60 Gew.-%, bevorzugt 10 bis 50 Gew.-% und ganz besonders bevorzugt 20 bis 40 Gew.-% Alkylacrylate mit 1 bis 10 Kohlenstoffatomen im Alkylrest, deren Alkylrest keine Doppelbindungen oder Heteroatome aufweist, umfassen.

Überraschende Vorteile können insbesondere durch Monomermischungen gemäß der zweiten Ausführungsform erzielt werden, die sich durch ein relativ geringes Gewichtsverhältnis von Alkylacrylaten mit 1 bis 10 Kohlenstoffatomen im Alkylrest zu Alkylmethacrylaten mit 1 bis 10 Kohlenstoffatomen im Alkylrest auszeichnen. Vorzugsweise kann das Gewichtsverhältnis von Alkylacrylat mit 1 bis 10 Kohlenstoffatomen im Alkylrest zu Alkylmethacrylat mit 1 bis 10 Kohlenstoffatomen im Alkylrest im Bereich von 1:50 bis 1:1, besonders bevorzugt 1:5 bis 2:3 liegen.

Monomermischungen gemäß der zweiten Ausführungsform eignen sich insbesondere zur Herstellung von Polymeren bzw. Beschichtungsmitteln, die sich für relativ feste Materialien, insbesondere für Holz, Metalle und Kunststoffe eignen.

Neben den zuvor dargelegten Monomeren kann eine erfindungsgemäße Monomermischung weitere Monomere enthalten, welche mit diesen copolymerisierbar sind. Zu diesen copolymerisierbaren Monomeren gehören unter anderem Monomere mit einer Säuregruppe, Estergruppen umfassenden Monomere A, die sich von den zuvor dargelegten (Meth)acrylaten mit mindestens einer Aldehydgruppe im Alkylrest und Alkyl(meth)acrylaten mit 1 bis 10 Kohlenstoffatomen im Alkylrest unterscheiden, Monomere mit einer Amino- oder Amidgruppe und Styrolmonomere.

Eine bevorzugte Gruppe von Comonomeren stellen Monomere mit einer Amino- oder Amidgruppe dar.

Zu diesen Monomeren gehören insbesondere Monomere mit einer primären Amidgruppe ($-CO-NH_2$) oder einer sekundären Amidgruppe (-CO-NHR), wie zum Beispiel (Meth)acrylamid, N-Methylolmethacrylamid, N,N-Dimethylaminopropyl(meth)acrylamid, Diacetonacrylamid, wobei Methacrylamid ganz besonders bevorzugt ist. Ferner gehören hierzu Monomere, die von Fettsäureamiden abgeleitet sind, wie zum Beispiel (Meth)acrylate, die sich von gesättigten Fettsäureamiden ableiten, wie Pentadecyloyloxy-2-ethyl-(meth)acrylsäureamid, Heptadecyloyloxy-2-ethyl-(meth)acrylsäureamid, (Meth)acryloyloxy-2-ethyl-laurinsäureamid, (Meth)acryloyloxy-2-ethyl-myristinsäureamid, (Meth)acryloyloxy-2-ethyl-palmitinsäureamid, (Meth)acryloyloxy-2-ethyl-stearinsäureamid, (Meth)acryloyloxy-2-propyl-laurinsäureamid, (Meth)acryloyloxy-2-propyl-myristinsäureamid, (Meth)acryloyloxy-2-propyl-palmitinsäureamid und (Meth)acryloyloxy-2-propyl-stearinsäureamid und (Meth)acrylate, die sich von ungesättigten Fettsäureamiden ableiten, wie Heptadecenyloyloxy-2-ethyl-(meth)acrylsäureamid, Heptadeca-dien-yloyloxy-2-ethyl-(meth)acrylsäureamid, Heptadeca-trien-yloyloxy-2-ethyl-(meth)acrylsäureamid, Heptadecenyloyloxy-2-ethyl-(meth)acrylsäureamid, (Meth)acryloyloxy-2-ethyl-palmitoleinsäuresäureamid, (Meth)acryloyloxy-2-ethyl-ölsäureamid, (Meth)acryloyloxy-2-ethyl-icosensäureamid, (Meth)acryloyloxy-2-ethyl-cetoleinsäureamid, (Meth)acryloyloxy-2-ethyl-erucasäureamid, (Meth)acryloyloxy-2-ethyl-linolsäureamid, (Meth)acryloyloxy-2-ethyl-linolensäureamid, (Meth)acryloyloxy-2-propylpalmitoleinsäuresäureamid, (Meth)acryloyloxy-2-propyl-ölsäureamid, (Meth)acryloyloxy-2-propyl-icosensäureamid, (Meth)acryloyloxy-2-propylcetoleinsäureamid, (Meth)acryloyloxy-2-propyl-erucasäureamid, (Meth)acryloyloxy-2-propyl-linolsäureamid und (Meth)acryloyloxy-2-propyl-linolensäureamid.

Hierbei sind insbesondere Monomere mit einer primären Amidgruppe bevorzugt.
Weiterhin sind Monomere mit einer Amidgruppe bevorzugt, die bis zu 20 Kohlenstoffatome, vorzugsweise bis zu 15 Kohlenstoffatome und besonders bevorzugt bis zu 10 Kohlenstoffatome aufweisen.

Weiterhin gehören hierzu Monomere mit einer primären Aminogruppe, wie zum Beispiel Aminoethyl(meth)acrylat, Aminopropyl(meth)acrylat, Aminopentyl(meth)acrylat und Aminohexadecyl(meth)acrylat; sekundären Aminogruppe, wie zum Beispiel N-Methylaminoethyl(meth)acrylat, N-Methylaminopropyl(meth)acrylat, N-Ethylaminopentyl(meth)acrylat und N-Butylaminohexadecyl(meth)acrylat; oder tertiären Aminogruppe, wie zum Beispiel N,N-Dimethylaminoethyl(meth)acrylat, N,N-Dimethylaminopropyl(meth)acrylat, N,N-Diethylaminopentyl(meth)acrylat und N,N-Dibutylaminohexadecyl(meth)acrylat.

Gemäß einer bevorzugten Ausführungsform sind insbesondere Monomere mit einer primären Amidgruppe ($-CO-NH_2$) oder einer sekundären Amidgruppe (-CO-NHR) bevorzugt. Beschichtungen, die aus Beschichtungszusammensetzungen mit Polymeren erhalten wurden, die Amidgruppen umfassen, zeigen gute mechanische Eigenschaften und eine hohe Lösungsmittelbeständigkeit, wobei diese Beschichtungszusammensetzungen eine hervorragende Haltbarkeit aufweisen.

Gemäß einem weiteren Aspekt sind Monomere mit einer Aminogruppe bevorzugt. Beschichtungen, die aus Beschichtungsmitteln mit Polymeren erhältlich sind, die Aminogruppen umfassen, zeigen eine herausragende Leistungsfähigkeit, wobei diese Leistungsfähigkeit insbesondere gute mechanische Eigenschaften und ausgezeichnete Resistenzen gegenüber unterschiedlichen Lösungsmitteln umfasst. Diese Leistungsfähigkeit wird bei vergleichsweise geringen Temperungstemperaturen und relativ kurzen Temperungszeiten erzielt. Hierbei können insbesondere Monomere mit einer sekundären Aminogruppe zu einer besonderen Leistungsfähigkeit, insbesondere bezüglich der mechanischen Eigenschaften und der Lösungsmittelresistenz führen. Monomere mit einer primären oder tertiären Aminogruppe zeichnen sich durch ein relativ ausgewogenes Verhältnis zwischen Haltbarkeit des Beschichtungsmittels und der Leistungsfähigkeit der Beschichtung aus.

Gemäß einem besonderen Aspekt der vorliegenden Erfindung kann eine Monomermischung 0,1 bis 10 Gew.-%, besonders bevorzugt 0,5 bis 5 Gew.-% Monomer mit einer Amino- oder Amidgruppe aufweisen, bezogen auf das Gesamtgewicht der Monomere.

Bei Monomermischungen zur Herstellung von Beschichtungsmitteln, die besonders für flexible Materialien, wie Textilien geeignet sind, können durch Verwendung von Monomeren mit einer Amino- oder Amidgruppe überraschende Vorteile hinsichtlich der Chemikalienbeständigkeit, insbesondere gegenüber einer Quellung in MIBK erzielt werden.

Bei einer Anwendung auf relativ festen Substraten, insbesondere Holz oder Metallen, zeigen Polymere bzw. Beschichtungsmittel, die von Monomeren mit einer Amino- oder Amidgruppe abgeleitet sind, überraschende Vorteile hinsichtlich der mechanischen Eigenschaften, insbesondere der Zugfestigkeit.

Überraschende Vorteile können insbesondere durch Monomermischungen erzielt werden, die Polyalkylenglykolmono(meth)acrylate enthalten. So zeigen Polymere, insbesondere Polymerdispersionen, die aus diesen Monomermischungen erhältlich sind, eine hervorragende Verarbeitbarkeit, insbesondere eine ausgezeichnete Scherstabilität.

Polyalkylenglykolmono(meth)acrylate sind Monomere, die neben einer (Meth)acrylat-Gruppe einen Polyalkylenglykol-Rest aufweisen. Die Herstellung dieser Monomere ist unter anderem in WO 2006/024538, eingereicht am 02.09.2005 beim Europäischen Patentamt mit der Anmeldenummer PCT/EP2005/009466; und WO 2005/000929, eingereicht am 20.05.2004 beim Amerikanischen Patentamt (USPTO) mit der Anmeldenummer PCT/US2004/015898; dargelegt, wobei zu Zwecken der Offenbarung auf diese Druckschriften verwiesen wird und die darin beschriebenen Polyalkylenglykolmono(meth)acrylate und Verfahren zu deren Herstellung in die vorliegende Anmeldung eingefügt werden. So können Polyalkylenglykolmono(meth)acrylate mit einer Hydroxygruppe durch Umsetzung von (Meth)acrylsäure mit Epoxiden erhalten werden. Weiterhin können Polyalkylenglykolmono(meth)acrylate durch Umesterung von Alkyl (meth)acrylaten mit Alkoxypolyalkylenglykolen, insbesondere Methoxypolyalkylenglykolen erhalten werden.

Das Gewichtsmittel des Molekulargewichts des Polyalkylenglykolmono(meth)acrylats liegt vorzugsweise im Bereich von 350 bis 20000 g/mol, besonders bevorzugt im Bereich 500 bis 10000 g/mol, gemessen gemäß GPC.

Zu den bevorzugten Polyalkylenglykolen zur Herstellung der Polyalkylenglykolmono(meth)acrylate gehören insbesondere Poly-$C_2$-$C_4$-alkylenglykolverbindungen. Unter Poly-$C_2$-$C_4$-alkylenglykolverbindungen, die verschiedentlich auch als Poly-$C_2$-$C_4$-alkylenoxide oder Poly(oxy-$C_2$-$C_4$-alkylen) verbindungen bezeichnet werden, versteht man oligomere bzw. makromolekulare Polyether mit mehreren, in der Regel wenigstens 3, häufig wenigstens 5 und insbesondere wenigstens 10 und in der Regel nicht mehr als 500, häufig nicht mehr als 400, z. B. 7 bis 300 und insbesondere 10 bis 200 Wiederholungseinheiten, die von $C_2$-$C_4$-Alkylenglykolen abgeleitet sind. Diese Verbindungen können linear oder verzweigt sein.

Bevorzugte Polyalkylenglykolmono(meth)acrylate lassen sich durch die allgemeine Formel (III) beschreiben:

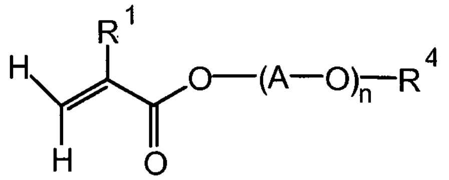

(III),

worin n die Anzahl der Wiederholungseinheiten angibt und in der Regel für eine Zahl im Bereich von 3 bis 500, insbesondere im Bereich von 5 bis 400, besonders bevorzugt im Bereich von 10 bis 300 und ganz besonderes bevorzugt im Bereich von 10 bis 200 steht, A für $C_2$-$C_4$-Alkylen wie 1,2-Ethandiyl, 1,3-Propandiyl, 1,2-Propandiyl, 1,2-Butandiyl oder 1,4-Butandiyl; $R^1$ für Wasserstoff oder Methyl und $R^4$ für Wasserstoff oder Alkyl mit vorzugsweise 1 bis 10 und insbesondere 1 bis 4 C-Atomen, Phenyl, Benzyl, Acyl (= C(O)-Alkyl) mit vorzugsweise 1 bis 10 C-Atomen, $SO_3H$-Gruppen oder $PO_3H_2$, insbesondere $C_1$-$C_{10}$-Alkyl und besonders bevorzugt $C_1$-$C_4$-Alkyl und speziell Methyl oder Ethyl steht.

Besonders bevorzugt einsetzbare (Poly-$C_2$-$C_4$-alkylenglykol)-mono(meth)acrylsäureester zeichnen sich dadurch aus, dass wenigstens 50 Gew.-%, vorzugsweise wenigstens 70 Gew.-%, insbesondere wenigstens 90 Gew.-% und speziell alle der Wiederholungseinheiten A-O in Formel (III) von Ethylenglykol bzw. von Ethylenoxid abgeleitet sind. Dementsprechend stehen vorzugsweise wenigstens 50 Gew.-%, insbesondere wenigstens 70 Gew.-%, ganz besonders bevorzugt wenigstens 90 Gew.-% und speziell alle der Einheiten A-O in Formel (III) für $CH_2$-$CH_2$-O. Gemäß einer weiteren bevorzugten Ausgestaltung der vorliegenden Erfindung können wenigstens 50 Gew.-%, vorzugsweise wenigstens 70 Gew.-%, insbesondere wenigstens 90 Gew.-% und speziell alle der Wiederholungseinheiten A-O in Formel (III) von Propylenglykol bzw. Propylenoxid abgeleitet sein.

Zu den bevorzugten Polyalkylenglykolmono(meth)acrylaten gehören insbesondere Alköxypolyalkylenglykolmono(meth)acrylate, die sich durch eine Alkylgruppe als Rest $R^4$ in obiger Formel (III) auszeichnen. Hierbei sind insbesondere Methoxypolyethylenglykolmono(meth)acrylate; auch als MPEG-(Meth)acrylate bezeichnet, besonders bevorzugt.

Besonders bevorzugt einzusetzende Polyalkylenglykolmono(meth)acrylate sind Methoxypolyethylenglykolmonomethacrylate mit der CAS-Nr. 26 915-72-0. Diese Methoxypolyethylenglykolmonomethacrylate weisen bevorzugt ein Zahlenmittel des Molekulargewichts im Bereich von 350 bis 5500 auf, so dass n in obiger Formel (III) vorzugsweise im Bereich von 6 bis 120 liegt. Diese Monomere können insbesondere unter den Handelsbezeichnungen Plex® 6850-0, Plex® 6969-0, Plex® 6968-0 und Plex® 6965-0 von Evonik Röhm GmbH kommerziell erworben werden.

Überraschende Vorteile lassen sich insbesondere durch Monomermischungen erzielen, die 0,1 bis 10 Gew.-%, besonders bevorzugt 1 bis 5 Gew.-% und ganz besonders bevorzugt 2 bis 3 Gew.-% Polyalkylenglykolmono(meth) acrylate, bezogen auf das Gewicht der Monomere in der Monomermischung, aufweisen.

Säuregruppen-haltige Monomere sind Verbindungen, die sich vorzugsweise radikalisch mit den zuvor dargelegten Monomeren copolymerisieren lassen. Hierzu gehören beispielsweise Monomere mit einer Sulfonsäuregruppe, wie zum Beispiel Vinylsulfonsäure; Monomere mit einer Phosphonsäuregruppe, wie zum Beispiel Vinylphosphonsäure und ungesättigte Carbonsäuren, wie zum Beispiel Methacrylsäure, Acrylsäure, Itaconsäure, Fumarsäure und Maleinsäure. Besonders bevorzugt sind Methacrylsäure und Acrylsäure. Die Säuregruppen-haltigen Monomere können einzeln oder als Mischung von zwei, drei oder mehr Säuregruppen-haltigen Monomeren eingesetzt werden.

Gemäß einer bevorzugten Ausführungsform der vorliegenden Erfindung kann eine Monomermischung 0,1 bis 10 Gew.-%, besonders bevorzugt 0,5 bis 5 Gew.-% Säure-gruppen-haltige Monomere aufweisen, bezogen auf das

Gesamtgewicht der Monomere.

Zu den bevorzugten Estergruppen umfassenden Monomere A gehören insbesondere (Meth)acrylate, die sich von den Monomeren gemäß Formel (I) und den Alkyl(meth)acrylaten mit 1 bis 10 Kohlenstoffatomen im Alkylrest unterscheiden, Fumarate, Maleate und/oder Vinylacetat.

Hierzu gehören beispielsweise (Meth)acrylate mit mindestens 11 Kohlenstoffatomen im Alkylrest, die sich von gesättigten Alkoholen ableiten, wie beispielsweise 2-tert.-Butylheptyl(meth)acrylat, Undecyl(meth)acrylat, 5-Methylundecyl(meth)acrylat, Dodecyl(meth)acrylat, 2-Methyldodecyl(meth)acrylat, Tridecyl(meth)acrylat, 5-Methyltridecyl(meth) acrylat, Tetradecyl(meth)acrylat, Pentadecyl(meth)acrylat, Hexadecyl(meth)acrylat, 2-Methylhexadecyl(meth)acrylat, Heptadecyl(meth)acrylat, 5-iso-Propylheptadecyl(meth)acrylat, 4-tert.-Butyloctadecyl(meth)acrylat, 5-Ethyloctadecyl (meth)acrylat, 3-iso-Propyloctadecyl(meth)acrylat, Octadecyl(meth)acrylat, Nonadecyl(meth)acrylat, Eicosyl(meth)acrylat, Cetyleicosyl(meth)acrylat, Stearyleicosyl(meth)acrylat, Docosyl(meth)acrylat und/oder Eicosyltetratriacontyl(meth) acrylat;

Cycloalkyl(meth)acrylate, wie 3-Vinylcyclohexyl(meth)acrylat, 2,4,5-Tri-t-butyl-3-vinylcyclohexyl(meth)acrylat, 2,3,4,5-Tetra-t-butylcyclohexyl(meth)acrylat;

heterocyclische (Meth)acrylate, wie 2 (1 Imidazolyl)ethyl(meth)acrylat, 2 (4 Morpholinyl)ethyl(meth)acrylat, 1 (2 Methacryloyloxyethyl)-2-pyrrolidon, 2-(3-Oxazolidinyl)ethylmethacrylat;

Nitrile der (Meth)acrylsäure und andere stickstoffhaltige Methacrylate, wie N-(Methacryloyloxyethyl)diisobutylketimin, N-(Methacryloyloxyethyl)dihexadecylketimin, Methacryloylamidoacetonitril, 2-Methacryloyloxyethylmethylcyanamid, Cyanomethylmethacrylat;

Aryl(meth)acrylate, wie Benzyl(meth)acrylat oder Phenyl(meth)acrylat, wobei die Arylreste jeweils unsubstituiert oder bis zu vierfach substituiert sein können;

(Meth)acrylate mit einer Hydroxygruppe im Alkylrest, insbesondere 2-Hydroxyethyl(meth)acrylat, vorzugsweise 2-Hydroxyethylmethacrylat (HEMA), Hydroxypropyl(meth)acrylat, beispielsweise 2-Hydroxypropyl(meth)acrylat und 3-Hydroxypropyl(meth)acrylat, vorzugsweise Hydroxypropylmethacrylat (HPMA), Hydroxybutyl(meth)acrylat, vorzugsweise Hydroxybutylmethacrylat (HBMA), 3,4-Dihydroxybutyl(meth)acrylat, 2,5-Dimethyl-1,6-hexandiol(meth)acrylat 1,10-Decandiol(meth)acrylat, Glycerinmono(meth)acrylat und

polyalkoxylierte Derivate der (Meth)acrylsäure, insbesondere Polypropylenglycolmono(meth)acrylat mit 2 bis 10, vorzugsweise 3 bis 6 Propylenoxideinheiten, vorzugsweise Polypropylenglycol-monomethacrylat mit ca. 5 Propylenoxideinheiten (PPM5), Polyethylenglycol-mono(meth)acrylat mit 2 bis 10, vorzugsweise 3 bis 6 Ethylenoxideinheiten, vorzugsweise Polyethylenglycol-monomethacrylat mit ca. 5 Ethylenoxideinheiten (PEM5), Polybutylenglycol-mono(meth) acrylat, Polyethylenglycolpolypropylenglycol-mono(meth)acrylat;

(Meth)acrylate, die zwei oder mehr (Meth)acryl-Gruppen aufweisen, Glycoldi(meth)acrylate, wie Ethylenglycoldi (meth)acrylat, Diethylenglycoldi(meth)acrylat, Triethylenglycoldi(meth)acrylat, Tetra- und Polyethylenglycoldi(meth) acrylat, 1,3- Butandiol(meth)acrylat, 1,4-Butandiol(meth)acrylat, 1,6-Hexandioldi(meth)acrylat, Glycerindi(meth)acrylat;

Dimethacrylate von ethoxyliertem Bisphenol A;

(Meth)acrylate mit drei oder mehr Doppelbindungen, wie z.B. Glycerintri(meth)acrylat, Trimethylolpropantri(meth)acrylat, Pentaerythrittetra(meth)acrylat und Dipentaerythritpenta(meth)acrylat;

Glycerincarbonatmethacrylat,

2-Carbamoyloxyethyl methacrylat.

Zu den Estergruppen umfassenden Monomeren A gehören darüber hinaus Vinylester, wie Vinylacetat, Vinylchlorid, Vinylversatat, Ethylenvinylacetat, Ethylenvinylchlorid;

Maleinsäurederivate, wie beispielsweise Maleinsäureanhydrid, Ester der Maleinsäure, beispielsweise Maleinsäuredimethylester, Methylmaleinsäureanhydrid; und Fumarsäurederivate, wie Fumarsäuredimethylester.

Eine weitere bvorzugte Gruppe von Comonomeren sind Styrolmonomere, wie zum Beispiel Styrol, substituierte Styrole mit einem Alkylsubstituenten in der Seitenkette, wie z. B. $\alpha$-Methylstyrol und $\alpha$-Ethylstyrol, substituierte Styrole mit einem Alkylsubstitutenten am Ring, wie Vinyltuluol und p-Methylstyrol, halogenierte Styrole, wie beispielsweise Monochlorstyrole, Dichlorstyrole, Tribromstyrole und Tetrabromstyrole.

Neben den zuvor dargelegten Monomeren können erfindungsgemäße Polymere, die durch die Polymerisation von Monomermischungen erhalten werden, weitere Monomere aufweisen. Hierzu gehören zum Beispiel Heterocyclische Vinylverbindungen, wie 2-Vinylpyridin, 3-Vinylpyridin, 2-Methyl-5-vinylpyridin, 3-Ethyl-4-vinylpyridin, 2,3-Dimethyl-5-vinylpyridin, Vinylpyrimidin, Vinylpiperidin, 9-Vinylcarbazol, 3-Vinylcarbazol, 4-Vinylcarbazol, 1-Vinylimidazol, 2-Methyl-1-vinylimidazol, N-Vinylpyrrolidon, 2-Vinylpyrrolidon, N-Vinylpyrrolidin, 3-Vinylpyrrolidin, N-Vinylcaprolactam, N-Vinylbutyrolactam, Vinyloxolan, Vinylfuran, Vinylthiophen, Vinylthiolan, Vinylthiazole und hydrierte Vinylthiazole, Vinyloxazole und hydrierte Vinyloxazole;

1-[2-[[2-Hydroxy-3-(2-propenyloxy)propyl]amino]ethyl]-2-imidazolidinon;

1-[2-[2-Hydroxy-3-(2-propen-1-yloxy)propoxy]ethyl]-2-imidazol id inon,

2-Methyl-N-[2-(2-oxo-1-imidazolidinyl)ethyl]-2-propenamid,

2-Methyl-,2-(2-oxo-1-imidazolidinyl)ethyl-2-propenester,

Trimethylolpropanoxetan,
1-(Butylamino)-3-(2-propen-1-yloxy)-2-propanol;
Maleinimid, Methylmaleinimid;
Vinyl- und Isoprenylether; und
Vinylhalogenide, wie beispielsweise Vinylchlorid, Vinylfluorid, Vinylidenchlorid und Vinylidenfluorid.

Gemäß der ersten Ausführungsform sind insbesondere Monomermischungen bevorzugt, d ie
30 bis 99 Gew.-% Alkylacrylat mit 1 bis 10 Kohlenstoffatomen im Alkylrest, dessen Alkylrest keine Doppelbindungen oder Heteroatome aufweist,
0,1 bis 10 Gew.-% (Meth)acrylat mit mindestens einer Aldehydgruppe im Alkylrest,
0,1 bis 10 Gew.-% (Meth)acrylmonomer mit einer Amino- oder Amidgruppe,
0 bis 60 Gew.-% Alkylmethacrylat mit 1 bis 10 Kohlenstoffatomen im Alkylrest und
0 bis 10 Gew.-% Polyalkylenglykolmono(meth)acrylat, jeweils bezogen auf das Gewicht der Monomeren, äufweisen.

Ferner sind Monomermischungen gemäß der ersten Ausführungsform besonders bevorzugt, die
30 bis 99 Gew.-% Alkylacrylat mit 1 bis 10 Kohlenstoffatomen im Alkylrest, dessen Alkylrest keine Doppelbindungen oder Heteroatome aufweist,
1 bis 8 Gew.-% (Meth)acrylat mit mindestens einer Aldehydgruppe im Alkylrest,
1 bis 5 Gew.-% (Meth)acrylmonomer mit einer Amino- oder Amidgruppe,
10 bis 45 Gew.-% Alkylmethacrylat mit 1 bis 10 Kohlenstoffatomen im Alkylrest und
1 bis 5 Gew.-% Polyalkylenglykolmono(meth)acrylat, jeweils bezogen auf das Gewicht der Monomeren, aufweisen.

Gemäß der zweiten Ausführungsform sind insbesondere Monomermischungen bevorzugt, die
30 bis 99 Gew.-% Alkylmethacrylat mit 1 bis 10 Kohlenstoffatomen im Alkylrest, dessen Alkylrest keine Doppelbindungen oder Heteroatome aufweist,
0,1 bis 10 Gew.-% (Meth)acrylat mit mindestens einer Aldehydgruppe im Alkylrest,
0,1 bis 10 Gew.-% (Meth)acrylmonomer mit einer Amino- oder Amidgruppe,
0 bis 60 Gew.-% Alkylacrylate mit 1 bis 10 Kohlenstoffatomen im Alkylrest und
0 bis 10 Gew.-% Polyalkylenglykolmono(meth)acrylat, jeweils bezogen auf das Gewicht der Monomeren, aufweisen.

Weiterhin sind Monomermischungen gemäß der zweiten Ausführungsform besonders bevorzugt, die
30 bis 99 Gew.-% Alkylmethacrylat mit 1 bis 10 Kohlenstoffatomen im Alkylrest, dessen Alkylrest keine Doppelbindungen oder Heteroatome aufweist,
1 bis 8 Gew.-% (Meth)acrylat mit mindestens einer Aldehydgruppe im Alkylrest,
1 bis 5 Gew.-% (Meth)acrylmonomer mit einer Amino- oder Amidgruppe,
10 bis 50 Gew.-% Alkylacrylate mit 1 bis 10 Kohlenstoffatomen im Alkylrest und
1 bis 5 Gew.-% Polyalkylenglykolmono(meth)acrylat, jeweils bezogen auf das Gewicht der Monomeren, aufweisen.

Darüber hinaus sind Monomermischungen bevorzugt, die einen sehr geringen Anteil an (Meth)acrylaten aufweisen, die zwei oder mehr Kohlenstoff-KohlenstoffDoppelbindungen aufweisen, die eine mit einer (Meth)acrylat-Gruppe identische Reaktivität aufweisen. Gemäß einer besonderen Abwandlung der vorliegenden Erfindung ist der Anteil an Verbindungen mit zwei oder mehr (Meth)acrylat-Gruppen vorzugsweise auf höchstens 5 Gew.-%, insbesondere höchstens 2 Gew.-%, insbesondere bevorzugt höchstens 1 Gew.-%, besonders bevorzugt höchstens 0,5 Gew.-% und ganz besonders bevorzugt höchstens 0,1 Gew.-% begrenzt, bezogen auf das Gesamtgewicht der Monomere.

Die Monomermischungen der vorliegenden Erfindung können insbesondere zur Herstellung oder zur Modifikation von Polymeren verwendet werden. Die Polymerisation kann durch jede bekannte Weise erfolgen. Hierzu gehören insbesondere die radikalische, kationische oder anionische Polymerisation, wobei auch Varianten dieser Polymerisationsverfahren, wie beispielsweise ATRP (=Atom Transfer Radical Polymerisation), NMP-Verfahren (Nitroxide Mediated Polymerization) oder RAFT (=Reversible Addition Fragmentation Chain Transfer) eingesetzt werden können.

Die hierdurch erhältlichen Polymere sind neu und daher ebenfalls Gegenstand der vorliegenden Erfindung. Die erfindungsgemäßen Polymere umfassen mindestens eine Einheit, die von einem (Meth)acrylat mit mindestens einer Aldehydgruppe im Alkylrest abgeleitet ist. Wie bereits beschrieben, können die erfindungsgemäßen Monomere durch radikalische Polymerisation umgesetzt werden. Daher ergibt sich der Begriff "Einheit" aus der Umsetzung einer Doppelbindung, wobei zwei kovalente Bindungen aufgebaut werden. Üblich werden diese Einheiten auch als Wiederholungseinheiten bezeichnet, falls zwei oder mehr dieser Einheiten in einem Polymer enthalten sind.

Die zuvor genannten Monomere bzw. Monomermischungen können beispielsweise durch Lösungspolymerisationen, Substanzpolymerisationen oder Emulsionspolymerisationen umgesetzt werden, wobei überraschende Vorteile durch eine radikalische Emulsionspolymerisation erzielt werden können.

Verfahren der Emulsionspolymerisation sind unter anderem in Ullmann's Encyclopedia of Industrial Chemistry, Fifth Edition dargelegt. Im Allgemeinen wird hierfür eine wässrige Phase hergestellt, die neben Wasser übliche Additive, insbesondere Emulgatoren und Schutzkolloide zur Stabilisierung der Emulsion umfassen kann.

Zu dieser wässrigen Phase werden anschließend Monomere hinzugegeben und in der wässrigen Phase polymerisiert. Bei Herstellung homogener Polymerteilchen kann hierbei eine Monomermischung über ein Zeitintervall kontinuierlich oder chargenweise zugegeben werden.

Die Emulsionspolymerisation kann beispielsweise als Mini- oder als Mikroemulsion ausgeführt werden, die näher in Chemistry and Technology of Emulsion Polymerisation, A.M. van Herk (editor), Blackwell Publishing, Oxford 2005 und J. O'Donnell, E.W. Kaler, Macromolecular Rapid Communications 2007, 28(14), 1445-1454 dargestellt werden. Eine Miniemulsion ist üblich durch die Verwendung von Costabilisatoren oder Quellmitteln gekennzeichnet, wobei vielfach langkettige Alkane oder Alkanole eingesetzt werden. Die Tröpfchengröße bei Miniemulsionen liegt vorzugsweise im Bereich von 0,05 bis 20 $\mu$m. Die Tröpfchengröße bei Mikroemulsionen liegt bevorzugt im Bereich unterhalb von 1 $\mu$m, wobei hierdurch Partikel unterhalb einer Größe von 50 nm erhalten werden können. Bei Mikroemulsionen werden vielfach zusätzliche Tenside, beispielsweise Hexanol oder ähnliche Verbindungen verwendet.

Das Dispergieren der monomerhaltigen Phase in der wässrigen Phase kann mit bekannten Mitteln erfolgen. Hierzu gehören insbesondere mechanische Verfahren sowie die Anwendung von Ultraschall.

Bei der Herstellung von homogenen Emulsionspolymerisaten kann vorzugsweise eine Monomermischung eingesetzt werden, die 0,1 bis 20 Gew.-%, besonders bevorzugt 1 bis 10 Gew.-%, insbesondere 2 bis 6 Gew.-% (Meth) acrylate mit mindestens einer Aldehydgruppe im Alkylrest umfasst.

Bei Herstellung von Kern-Schale-Polymeren kann die Zusammensetzung der Monomermischung schrittweise geändert werden, wobei vor Änderung der Zusammensetzung die Polymerisation vorzugsweise bis zu einem Umsatz von mindestens 80 Gew.-%, besonders bevorzugt mindestens 95 Gew.-%, jeweils bezogen auf das Gesamtgewicht der eingesetzten Monomermischung, polymerisiert wird. Kern-Schale-Polymer steht hier für ein Polymerisat, das durch eine zwei- oder mehrstufige Emulsionspolymerisation hergestellt wurde, ohne dass der Kern-Schale-Aufbau beispielsweise elektronenmikroskopisch gezeigt wurde. Die Verfolgung des Reaktionsfortschrittes der Polymerisation in jeden Schritt kann auf bekannte Weise, beispielsweise gravimetrisch oder mittels Gaschromatographie erfolgen.

Die Monomerzusammensetzung zur Herstellung des Kerns umfasst vorzugsweise 50 bis 100 Gew.-% (Meth)acrylate, wobei besonders bevorzugt eine Mischung von Acrylaten und Methacrylaten eingesetzt wird. Gemäß einem besonderen Aspekt der vorliegenden Erfindung kann das Gewichtsverhältnis von Acrylaten zu Methacrylaten im Kern größer oder gleich 1, besonders bevorzugt größer oder gleich 2 betragen. Nach der Herstellung des Kerns kann auf diesen vorzugsweise eine Monomermischung aufgepfropft oder auf den Kern polymerisiert werden, die 1 bis 40 Gew.-%, besonders bevorzugt 2 bis 20 Gew.-%, insbesondere 3 - 10 Gew.-% (Meth)acrylate mit mindestens einer Aldehydgruppe im Alkylrest umfasst.

Die Emulsionspolymerisation wird vorzugsweise bei einer Temperatur im Bereich von 0 bis 120 °C, besonders bevorzugt im Bereich von 30 bis 100 °C durchgeführt. Dabei haben sich Polymersiationstemperaturen im Bereich von größer 60 bis kleiner 90 °C, zweckmäßigerweise im Bereich von größer 70 bis kleiner 85 °C, vorzugsweise im Bereich von größer 75 bis kleiner 85 °C, als ganz besonders günstig erwiesen.

Die Initiierung der Polymerisation erfolgt mit den für die Emulsionspolymerisation gebräuchlichen Initiatoren. Geeignete organische Initiatoren sind beispielsweise Hydroperoxide, wie tert.-Butyl-Hydroperoxid oder Cumolhydroperoxid. Geeignete anorganische Initiatoren sind Wasserstoffperoxid sowie die Alkalimetall- und die Ammoniumsalze der Peroxodischwefelsäure, insbesondere Ammonium, Natrium- und Kaliumperoxodisulfat. Geeignete Redox-Initiatorsysteme sind beispielsweise Kombinationen von tertiären Aminen mit Peroxiden oder Natriumdisulfit und Alkalimetall- und die Ammoniumsalze der Peroxodischwefelsäure, insbesondere Natrium-und Kaliumperoxodisulfat. Weitere Details können der Fachliteratur, insbesondere H. Rauch-Puntigam, Th. Völker, "Acryl- und Methacrylverbindungen", Springer, Heidelberg, 1967 oder Kirk-Othmer, Encyclopedia of Chemical Technology, Vol. 1, Seiten 386ff, J. Wiley, New York, 1978 entnommen werden. Im Rahmen der vorliegenden Erfindung ist der Einsatz von organischen und/oder anorganischen Initiatoren besonders bevorzugt.

Die genannten Initiatoren können sowohl einzeln als auch in Mischung verwendet werden. Sie werden vorzugsweise in einer Menge von 0,05 bis 3,0 Gew.-%, bezogen auf das Gesamtgewicht der Monomere der jeweiligen Stufe, eingesetzt. Man kann auch bevorzugt die Polymerisation mit einem Gemisch verschiedener Polymerisationsinitiatoren unterschiedlicher Halbwertzeit durchführen, um den Radikalstrom im Verlauf der Polymerisation sowie bei verschiedenen Polymerisationstemperaturen konstant zu halten.

Die Stabilisierung des Ansatzes erfolgt vorzugsweise mittels Emulgatoren und/oder Schutzkolloiden. Bevorzugt wird die Emulsion durch Emulgatoren stabilisiert, um eine niedrige Dispersionsviskosität zu erhalten. Die Gesamtmenge an Emulgator beträgt vorzugsweise 0,1 bis 15 Gew.-%, insbesondere 1 bis 10 Gew.-% und besonders bevorzugt 2 bis 5 Gew.-%, bezogen auf das Gesamtgewicht der eingesetzten Monomere. Gemäß einem besonderen Aspekt der vorliegenden Erfindung kann ein Teil der Emulgatoren während der Polymerisation zugegeben werden.

Besonders geeignete Emulgatoren sind anionische oder nichtionische Emulgatoren oder deren Mischungen, insbesondere

- Alkylsulfate, vorzugsweise solche mit 8 bis 18 Kohlenstoffatomen im Alkylrest, Alkyl- und Alkylarylethersulfate mit 8 bis 18 Kohlenstoffatomen im Alkylrest und 1 bis 50 Ethylenoxideinheiten;
- Sulfonate, vorzugsweise Alkylsulfonate mit 8 bis 18 Kohlenstoffatomen im Alkylrest, Alkylarylsulfonate mit 8 bis 18

Kohlenstoffatomen im Alkylrest, Ester und Halbester der Sulfobernsteinsäure mit einwertigen Alkoholen oder Alkylphenolen mit 4 bis 15 Kohlenstoffatomen im Alkylrest; gegebenenfalls können diese Alkohole oder Alkylphenole auch mit 1 bis 40 Ethylenoxideinheiten ethoxyliert sein;

- Phosphorsäureteilester und deren Alkali- und Ammoniumsalze, vorzugsweise Alkyl- und Alkylarylphosphate mit 8 bis 20 Kohlenstoffatomen im Alkyl- bzw. Alkylarylrest und 1 bis 5 Ethylenoxideinheiten;
- Alkylpolyglykolether, vorzugsweise mit 8 bis 20 Kohlenstoffatomen im Alkylrest und 8 bis 40 Ethylenoxideinheiten;
- Alkylarylpolyglykolether, vorzugsweise mit 8 bis 20 Kohlenstoffatomen im Alkyl- bzw. Alkylarylrest und 8 bis 40 Ethylenoxideinheiten;
- Ethylenoxid/Propylenoxid-Copolymere, vorzugsweise Blockcopolymere, günstigerweise mit 8 bis 40 Ethylenoxid- bzw. Propylenoxideinheiten.

Zu den besonders bevorzugten anionische Emulgatoren zählen insbesondere Fettalkoholethersulfate, Diisooctylsulfosuccinat, Laurylsulfat, C15-Paraffinsulfonat, wobei diese Verbindungen im Allgemeinen als Alkalimetallsalz, insbesondere als Natriumsalz eingesetzt werden können. Diese Verbindungen können insbesondere unter den Handelsbezeichnungen Disponil® FES 32, Aerosol® OT 75, Texapon® K1296 und Statexan® K1 von den Firmen Cognis GmbH, Cytec Industries, Inc. und Bayer AG kommerziell erhalten werden.

Zweckmäßige nichtionische Emulgatoren sind unter anderem tert-Octylphenolethoxylat mit 30 Ethylenoxideinheiten und Fettalkoholpolyethylenglykolether, die bevorzugt 8 bis 20 Kohlenstoffatome im Alkylrest und 8 bis 40 Ethylenoxideinheiten aufweisen. Diese Emulgatoren sind unter den Handelsbezeichnungen Triton® X 305 (Fluka), Tergitol® 15-S-7 (Sigma-Aldrich Co.), Marlipal® 1618/25 (Sasol Germany) und Marlipal® O 13/400 (Sasol Germany) kommerziell erhältlich.

Bevorzugt können Gemische aus anionischem Emulgator und nichtionischen Emulgator eingesetzt werden. Zweckmäßig kann das Gewichtsverhältnis von anionischem Emulgator zu nichtionischem Emulgator im Bereich von 20:1 bis 1:20, bevorzugt 2:1 bis 1:10 und besonders bevorzugt 1:1 bis 1:5 liegen. Dabei haben sich Gemische, die ein Sulfat, insbesondere ein Fettalkoholethersulfat, ein Laurylsulfat, oder ein Sulfonat, insbesondere ein Diisooctylsulfosuccinat oder ein Paraffinsulfonat als anionischen Emulgator und ein Alkylphenolethoxylat oder ein Fettalkoholpolyethylenglykolether, die jeweils bevorzugt 8 bis 20 Kohlenstoffatome im Alkylrest und 8 bis 40 Ethylenoxideinheiten aufweisen, als nichtionischem Emulgator ganz besonders bewährt.

Gegebenenfalls können die Emulgatoren auch in Mischung mit Schutzkolloiden eingesetzt werden. Geeignete Schutzkolloide umfassen u. a. teilverseifte Polyvinylacetate, Polyvinylpyrrolidone, Carboxymethyl-, Methyl-, Hydroxyethyl-, Hydroxypropyl-Cellulose, Stärken, Proteine, Poly(meth)acrylsäure, Poly(meth)acrylamid, Polyvinylsulfonsäuren, Melaminformaldehydsulfonate, Naphthalinformaldehydsulfonate, Styrol-Maleinsäure- und Vinylethermaleinsäure-Copolymere. Falls Schutzkolloide eingesetzt werden, erfolgt dies vorzugsweise in einer Menge von 0,01 bis 1,0 Gew.-%, bezogen auf die Gesamtmenge der Monomere. Die Schutzkolloide können vor dem Start der Polymerisation vorgelegt oder zudosiert werden. Der Initiator kann vorgelegt oder zudosiert werden. Weiterhin ist es auch möglich, einen Teil des Initiators vorzulegen und den Rest zuzudosieren.

Bevorzugt wird die Polymerisation durch Erhitzen des Ansatzes auf die Polymerisationstemperatur und Vorlage und/oder Zudosierung des Initiators, vorzugsweise in wässriger Lösung, gestartet. Dabei kann ein Teil der Monomeren im Reaktor vorgelegt und der Rest über einen bestimmten Zeitraum zudosiert werden. In der Regel ist es vorteilhaft, den im Reaktor vorgelegten Teil der Monomeren zu polymerisieren und erst dann mit dem Zulauf zu beginnen. Alternativ zur Vorlage einer definierten Monomermenge kann der Zulauf für einige Minuten unterbrochen werden, nachdem z.B. 1 - 5 % der Monomeren zudosiert sind. Die Dosierungen von Emulgator und Monomeren können separat durchgeführt werden oder vorzugsweise als Gemisch, insbesondere als Emulsion in Wasser.

Die Emulsionspolymerisation kann in einem breiten pH-Bereich durchgeführt werden. Vorzugsweise liegt er zwischen 2 und 9. In einer besonderen Ausführungsform wird die Polymerisation bei pH-Werten zwischen 4 und 8, insbesondere zwischen 6 und 8 durchgeführt. Ebenso kann die Dispersion nach der Polymerisation auf einen für die Anwendung bevorzugten pH-Bereich eingestellt werden. Für pigmentierte Beschichtungssysteme liegt der Bereich im Allgemeinen bei 8 - 9 oder darüber.

Zur Beschleunigung einer erwünschten Selbstvernetzung kann der pH-Wert der Dispersion weiterhin auf einen Wert im Bereich von 1 bis 4, besonders bevorzugt 1,5 bis 3 eingestellt werden. Dies kann vorzugsweise kurz vor der Applikation der Dispersion erfolgen. Darüber hinaus können der Dispersion zur Katalyse der Selbstvernetzung Katalysatoren zugegeben werden, wobei dies vorzugsweise kurz vor der Anwendung der Beschichtungsmittel erfolgen sollte. Zu diesen Katalysatoren gehören unter anderem Alkalicarbonate, Alkalicyanide, Natriumacetat, verdünnte Laugen und verdünnte Salzsäure.

Zur Verbesserung der Haltbarkeit der Beschichtungsmittel, insbesondere zur Vermeidung einer vorzeitigen Selbstvernetzung, kann der pH-Wert um den Neutralpunkt, d.h. im Bereich von 6 bis 9 eingestellt werden.

Das Molekulargewicht der Polymeren ist in weiten Grenzen zunächst unkritisch. Sind besonders harte und lösungsmittelbeständige Beschichtungsmittel mit guten mechanischen Eigenschaften gewünscht, so kann ein möglichst

hohes Molekulargewicht hilfreich sein. Bevorzugte Emulsionspolymere mit einem hohen Anteil an Polymeren, die in THF unlöslich sind, können auf die zuvor dargelegte Weise erhalten werden. Die Reaktionsparameter, um ein hohes Molekulargewicht zu erhalten, sind bekannt. So kann hierbei insbesondere auf die Verwendung von Molekulargewichtsreglern verzichtet werden.

Beschichtungsmittel, die sich besonders gut und einfach verarbeiten lassen, können auch Polymere mit einem geringeren Molekulargewicht aufweisen, wobei die Lösungsmittelbeständigkeit und die Härte dieser Beschichtungen ein relativ hohes Niveau erreicht. Vorzugsweise können diese Polymere mit einer besonders guten Verarbeitbarkeit ein Molekulargewicht unter 1 000 000 g/mol, bevorzugt unter 500 000 g/mol und besonders bevorzugt unter 250 000 g/mol aufweisen. Das Molekulargewicht kann mittels Gelpermeationschromatographie (GPC) gegen einen PMMA-Standard bestimmt werden.

Polymere, insbesondere Emulsionspolymere, mit einem geringen Molekulargewicht können durch die Zugabe von Molekulargewichtsreglern in die Reaktionsmischung vor oder während der Polymerisation erhalten werden. Hierzu können schwefelfreie Molekulargewichtsregler und/oder schwefelhaltige Molekulargewichtsregler eingesetzt werden.

Zu den schwefelfreien Molekulargewichtsreglern gehören beispielsweise, ohne dass hierdurch eine Einschränkung erfolgen soll, dimeres $\alpha$-Methylstyrol (2,4 Diphenyl-4-methyl-1-penten), Enolether aliphatischer und/oder cycloaliphatischer Aldehyde, Terpene, $\beta$-Terpinen, Terpinolen, 1,4-Cyclohexadien, 1,4-Dihydronaphthalin, 1,4,5,8-Tetrahydronaphthalin, 2,5-Dihydrofuran, 2,5-Dimethylfuran und/oder 3,6-Dihydro-2H-pyran, bevorzugt ist dimeres $\alpha$-Methylstyrol.

Als schwefelhaltige Molekulargewichtsregler können vorzugsweise Mercaptoverbindungen, Dialkylsulfide, Dialkyldisulfide und/oder Diarylsulfide eingesetzt werden. Folgende Polymerisationsregler werden beispielhaft genannt: Di-n-butylsulfid, Di-n-octylsulfid, Diphenylsulfid, Thiodiglykol, Ethylthioethanol, Diisopropyldisulfid, Di-n-butyl-disulfid, Di-n-hexyldisulfid, Diacetyldisulfid, Diethanolsulfid, Di-t-butyltrisulfid und Dimethylsulfoxid. Bevorzugt als Molekulargewichtsreglern eingesetzte Verbindungen sind Mercaptoverbindungen, Dialkylsulfide, Dialkyldisulfide und/oder Diarylsulfide. Beispiele für diese Verbindungen sind Ethylthioglykolat, 2-Ethylhexylthioglycolat, Cystein, 2-Mercaptoethanol, 1, 3-Mercaptopropanol, 3-Mercaptopropan-1,2-diol, 1,4-Mercaptobutanol, Mercaptoessigsäure, 3-Mercaptopropionsäure, Mercaptobernsteinsäure, Thioglycerin, Thioessigsäure, Thioharnstoff und Alkylmercaptane wie n-Butylmercaptan, n-Hexylmercaptan oder n-Dodecylmercaptan. Besonders bevorzugt eingesetzte Polymerisationsregler sind Mercaptoalkohole und Mercaptocarbonsäuren.

Die Molekulargewichtsregler werden vorzugsweise in Mengen von 0,05 bis 10, besonders bevorzugt 0,1 bis 5 Gew.-%, bezogen auf die bei der Polymerisation eingesetzten Monomeren, verwendet. Bei der Polymerisation können selbstverständlich auch Mischungen von Polymerisationsreglern angewendet werden.

Darüber hinaus können Polymerisationen unter Verwendung von Molekulargewichtsreglern zur Verringerung der Mindest-Filmbildungs-Temperatur (MFT) der hierdurch erhältlichen Polymere eingesetzt werden. Gemäß dieser bevorzugten Ausführungsform kann der Anteil an Molekulargewichtsreglern so bemessen werden, dass die Polymere bzw. die erfindungsgemäßen Beschichtungsmittel eine Mindest-Filmbildungs-Temperatur (MFT) von höchstens 60°C, besonders bevorzugt höchstens 50°C und ganz besonders bevorzugt höchstens 40°C aufweisen, die gemäß DIN ISO 2115 gemessen werden kann. Je höher der Anteil an Molekulargewichtsregler, desto geringer die Mindest-Filmbildungs-Temperatur.

Die Einstellung der Teilchenradien kann unter anderem über den Anteil an Emulgatoren beeinflusst werden. Je höher dieser Anteil, insbesondere zu Beginn der Polymerisation, desto kleinere Partikel werden erhalten.

Die gemäß dem zuvor beschriebenen Verfahren erhältlichen Polymere, insbesondere die bevorzugt erhältlichen Emulsionspolymere stellen einen weiteren Gegenstand der vorliegenden Erfindung dar.

Vorzugsweise ist das Emulsionspolymer unvernetzt oder so gering vernetzt, dass der in Tetrahydrofuran (THF) bei 20°C lösliche Anteil über 60 Gew.-% bezogen auf das Gewicht des Emulsionspolymeren liegt. In einer weiteren, bevorzugten Ausführungsform kann das Emulsionspolymer einen Anteil von 2 bis 60 Gew.-%, besonders bevorzugt 10 bis 50 Gew.-% und ganz besonders bevorzugt 20 bis 40 Gew.-% aufweisen, bezogen auf das Gewicht des Emulsionspolymeren, der in THF bei 20°C löslich ist. Zur Bestimmung des löslichen Anteils wird eine getrocknete Probe des Polymerisats in einer 200fachen Menge an Lösungsmittel, bezogen auf das Gewicht der Probe, bei 20°C für 4 h gelagert. Hierbei wird die Trocknung so vorgenommen, dass möglichst keine Selbstvernetzung eintritt. Dies kann beispielsweise durch eine Gefriertrocknung erfolgen. Nach der Lagerung wird die Lösung von dem unlöslichen Anteil, beispielsweise durch Filtration getrennt. Nach dem Verdampfen des Lösungsmittels wird das Gewicht des Rückstands bestimmt. Beispielsweise kann eine 0,5 g Probe eines unter Vakuum getrockneten Emulsionspolymeren in 150 ml THF 4 Stunden gelagert werden.

Der Teilchenradius der Emulsionspolymere kann in einem weiten Bereich liegen. So können insbesondere Emulsionspolymere mit einem Teilchenradius im Bereich von 10 bis 500 nm, bevorzugt 10 bis 100 nm, besonders bevorzugt 20 bis 60 nm eingesetzt werden. Insbesondere Teilchenradien unter 50 nm können vorteilhaft für die Filmbildung und die Beschichtungseigenschaften sein. Der Radius der Teilchen kann durch PCS (Photon Correlation Spektroscopy) bestimmt werden, wobei sich die angegebenen Daten auf den r50-Wert beziehen (50% der Teilchen sind

kleiner, 50% sind größer). Hierzu kann beispielsweise ein Beckman Coulter N5 Submicron Particle Size Analyzer eingesetzt werden.

Die Glasübergangstemperatur des erfindungsgemäßen Polymeren liegt vorzugsweise im Bereich von -60°C bis 100°C, insbesondere bevorzugt -30°C bis 70°C besonders bevorzugt im Bereich von -20 bis 40°C und ganz besonders bevorzugt im Bereich von 0 bis 25°C. Die Glasübergangstemperatur kann über die Art und den Anteil der zur Herstellung des Polymeren verwendeten Monomere beeinflusst werden. Dabei kann die Glasübergangstemperatur Tg des Polymerisates in bekannter Weise mittels Differential Scanning Calorimetry (DSC), insbesondere gemäß DIN EN ISO 11357 ermittelt werden. Vorzugsweise kann die Glasübergangstemperatur als Mittelpunkt der Glasstufe der zweiten Aufheizkurve mit einer Aufheizrate von 10°C pro Minute bestimmt werden: Weiterhin kann die Glasübergangstemperatur Tg auch mittels der Fox-Gleichung näherungsweise voraus berechnet werden. Nach Fox T. G., Bull. Am. Physics Soc. 1, 3,Seite 123 (1956) gilt:

$$\frac{1}{Tg} = \frac{x_1}{Tg_1} + \frac{x_2}{Tg_2} + \ldots + \frac{x_n}{Tg_n}$$

wobei $x_n$ für den Massebruch (Gew.-%/100) des Monomeren n steht und $Tg_n$ die Glasübergangstemperatur in Kelvin des Homopolymeren des Monomeren n bezeichnet. Weitere hilfreiche Hinweise kann der Fachmann dem Polymer Handbook 2nd Edition, J. Wiley & Sons, New York (1975) entnehmen, welche Tg-Werte für die geläufigsten Homopolymerisate angibt. Hierbei kann das Polymere ein oder mehrere unterschiedliche Glasübergangstemperaturen aufweisen. Diese Angaben gelten daher für ein Segment, das durch Polymerisation mindestens eines (Meth)acrylats mit mindestens einer Aldehydgruppe im Alkylrest, vorzugsweise einer erfindungsgemäßen Monomermischung erhältlich ist.

Die Architektur des Polymeren ist für viele Anwendungen und Eigenschaften nicht kritisch. Dementsprechend können die Polymere, insbesondere die Emulsionspolymere statistische Copolymere, Gradienten-Copolymere, Blockcopolymere und/oder Pfropfcopolymere darstellen. Blockcopolymere bzw. Gradienten-Copolymere kann man beispielsweise dadurch erhalten, dass man die Monomerenzusammensetzung während des Kettenwachstums diskontinuierlich verändert. Gemäß einem bevorzugten Aspekt der vorliegenden Erfindung ist das Emulsionspolymer ein statistisches Copolymer, bei welchem die Monomerzusammensetzung über die Polymerisation im Wesentlichen konstant ist. Da die Monomere jedoch unterschiedliche Copolymerisationsparameter aufweisen können, kann die genaue Zusammensetzung über die Polymerkette des Polymers schwanken.

Das Polymer kann ein homogenes Polymer darstellen, welches beispielsweise in einer wässrigen Dispersion Teilchen mit einer gleich bleibenden Zusammensetzung bildet. In diesem Fall kann das Polymer, welches bevorzugt ein Emulsionspolymer darstellt, aus einem oder mehrere Segmenten bestehen, die durch Polymerisation einer erfindungsgemäßen Monomermischung erhältlich sind.

Gemäß einer weiteren Ausführungsform kann das Emulsionspolymer ein Kern-Schale-Polymer darstellen, welches eine, zwei, drei oder mehr Schalen aufweisen kann. Hierbei bildet das Segment, welches durch Polymerisation der erfindungsgemäßen Monomermischung erhältlich ist, vorzugsweise die äußerste Schale des Kern-Schale-Polymers. Die Schale kann über kovalente Bindungen mit dem Kern oder den inneren Schalen verbunden sein. Weiterhin kann die Schale auch auf den Kern oder eine innere Schale polymerisiert werden. In dieser Ausführungsform kann das Segment, welches unter anderem durch Polymerisation der erfindungsgemäßen Monomermischung erhältlich ist, vielfach durch geeignete Lösungsmittel vom Kern getrennt und isoliert werden.

Vorzugsweise kann das Gewichtsverhältnis von Segment, welches durch Polymerisation der erfindungsgemäßen Monomermischung erhältlich ist, zu Kern im Bereich von 6:1 bis 1:6 liegen. Für den Fall, dass die Glastemperatur des Kerns höher liegt als die der Schale ist ein Verhältnis 6:1 bis 2:1, im umgekehrten Fall von 1:1 bis 1:5 besonders bevorzugt.

Der Kern kann vorzugsweise aus Polymeren gebildet werden, die 50 bis 100 Gew.-%, vorzugsweise 60 bis 90 Gew.-% Einheiten umfasst, die von (Meth)acrylaten abgeleitet sind. Bevorzugt sind hierbei Ester der (Meth)acrylsäure, deren Alkoholrest vorzugsweise 1 bis 30 Kohlenstoffatome, besonders bevorzugt 1 bis 20 Kohlenstoffatome und ganz besonders bevorzugt 1 bis 10 Kohlenstoffatome umfasst. Hierzu gehören insbesondere (Meth)acrylate, die sich von gesättigten Alkoholen ableiten, wie Methyl(meth)acrylat, Ethyl(meth)acrylat, n-Propyl(meth)acrylat, iso-Propyl(meth) acrylat, n-Butyl(meth)acrylat, tert.-Butyl(meth)acrylat und Pentyl(meth)acrylat, Hexyl(meth)acrylat.

Gemäß einer besonderen Ausgestaltung der vorliegenden Erfindung kann zur Herstellung des Kerns eine Mischung eingesetzt werden, die Methacrylate und Acrylate umfasst. So können insbesondere Mischungen von Methylmethacrylat und Acrylaten mit 2 bis 10, vorzugsweise 2 bis 8 Kohlenstoffatomen, wie Ethylacrylat, Butylacrylat, Hexylacrylat und Ethylhexylacrylat verwendet werden. Von Interesse sind insbesondere Monomermischungen zur Herstellung des Kerns bzw. einer der inneren Schalen, falls das Kern-Schale-Polymer mehr als eine Schale aufweist, die mindestens 30 Gew.-%, besonders bevorzugt mindestens 50 Gew.-% und ganz besonders bevorzugt mindestens 60

Gew.-% an Acrylaten mit 2 bis 10 Kohlenstoffatomen aufweisen, bezogen auf das Gesamtgewicht der Monomeren zur Herstellung des Kerns oder mindestens einer der inneren Schalen.

Darüber hinaus können die Polymere des Kerns die zuvor dargelegten Comonomere umfassen. Gemäß einer bevorzugten Abwandlung kann der Kern vernetzt sein. Diese Vernetzung kann durch die Verwendung von Monomeren mit zwei, drei oder mehr radikalisch polymerisierbaren Doppelbindungen erzielt werden.

Die durch Polymerisation einer erfindungsgemäßen Monomermischung erhältliche Schale eines Emulsionspolymeren der vorliegenden Erfindung kann bevorzugt 2 bis 8 Gew.-% Einheiten umfassen, die von (Meth)acrylate mit mindestens einer Aldehydgruppe im Alkylrest abgeleitet sind.

Gemäß einem besonderen Aspekt kann der Kern vorzugsweise eine Glasübergangstemperatur im Bereich von -30 bis 200°C, insbesondere im Bereich von -20 bis 150°C aufweisen. Besonders bevorzugt ist eine Glasübergangstemperatur von > 50 °C, insbesondere > 100 °C. Die Schale des erfindungsgemäßen Emulsionspolymers, die vorzugsweise durch Polymerisation der erfindungsgemäßen Monomermischung erhältlich ist, kann vorzugsweise eine Glasübergangstemperatur im Bereich von -60°C bis 100°C, insbesondere bevorzugt -30°C bis 70°C besonders bevorzugt im Bereich von - 20 bis 40°C und ganz besonders bevorzugt im Bereich von 0 bis 25 °C aufweisen. Gemäß einem besonderen Aspekt der vorliegenden Erfindung-kann die Glasübergangstemperatur des Kerns größer sein als die Glasübergangstemperatur der Schale. Zweckmäßig kann die Glasübergangstemperatur des Kerns mindestens 10°C, bevorzugt mindestens 20°C oberhalb der Glasübergangstemperatur der Schale liegen. Die Glasübergangstemperatur kann hierbei gemäß den zuvor dargelegten Verfahren, vorzugsweise gemäß DSC bestimmt werden.

Die durch Polymerisation von einer erfindungsgemäßen Monomermischung erhältlichen Polymere können isoliert werden. Gemäß einer besonderen Ausgestaltung der vorliegenden Erfindung können die durch Emulsionspolymerisation erhältlichen Dispersionen als solche als Beschichtungsmittel eingesetzt werden.

Beschichtungsmittel, die die zuvor dargelegten Polymere oder Verbindungen umfassen, die durch Reaktionen mit den zuvor dargelegten Monomermischungen erhältlich sind, sind ebenfalls Gegenstand der vorliegenden Erfindung. Beschichtungsmittel sind Zusammensetzungen, die zur Beschichtung von Substraten geeignet sind. Die erfindungsgemäßen Beschichtungsmittel sind durch Vernetzungsmittel vernetzbar. Weiterhin zeigen bevorzugte Beschichtungsmittel die Neigung einer Selbstvernetzung. Eine Selbstvernetzung kann insbesondere durch Temperung der Filme bei Temperaturen über 40 °C, bevorzugt über 60°C, besonders bevorzugt über 100°C erzielt werden.

Die Zeit, über die die Filme vernetzt werden, ist beispielsweise von dem pH-Wert des Films und von der Art und Menge der Aldehydgruppen-tragenden Wiederholungseinheiten und von der gewünschten mechanischen Festigkeit der Beschichtung abhängig.

Von besonderem Interesse sind insbesondere Verfahren, bei denen die Selbstvernetzung über eine Dauer im Bereich von 10 Sekunden bis 120 Minuten, besonders bevorzugt im Bereich von 1 Minute bis 30 Minuten durchgeführt wird. Vernetzte Filme zeichnen sich vielfach durch eine hohe Lösungsmittelbeständigkeit und hervorragende mechanische Eigenschaften aus.

Beschichtungsmittel bzw. Polymere, die Einheiten aufweisen, die von Monomeren mit einer Amino- oder Amidgruppe abgeleitet sind, zeigen überraschende Vorteile hinsichtlich der Chemikalienbeständigkeit und der mechanischen Eigenschaften, insbesondere der Zugfestigkeit nach einer Temperung der aus diesen Beschichtungsmitteln bzw. Polymeren erhaltenen Beschichtungen. So können die Chemikalienbeständigkeit und die mechanischen Eigenschaften in einem unerwarteten Maß durch eine Temperung verbessert werden.

Gemäß einer bevorzugten Ausführungsform kann die Selbstvernetzung so durchgeführt werden, dass der Umsatz vorzugsweise mindestens 5 %, besonders bevorzugt mindestens 10 %, insbesondere bevorzugt mindestens 20 % und ganz besonders bevorzugt mindestens 50 %, bezogen auf den Anteil der eingesetzten Aldehydgruppen, beträgt, um eine hervorragende Beschichtung zu erhalten. Es kann hierbei angenommen werden, dass in einem ersten Schritt ein Aldol gebildet wird.

Darüber hinaus sind insbesondere Varianten bevorzugt, bei denen die Selbstvernetzung bei einer erhöhten Temperatur durchgeführt wird, so dass eine Wasserabspaltung eintritt, die zu einer Bildung von Doppelbindungen führt. Überraschend können hierdurch besonders lösungsmittelbeständige und mechanisch stabile Beschichtungen erhalten werden.

Die Haltbarkeit der erfindungsgemäßen Beschichtungsmittel kann insbesondere durch eine Lagerung bei einer relativ tiefen Temperatur verbessert werden.

Vorzugsweise umfasst das Beschichtungsmittel nur geringen Mengen an umweltgefährdenden Lösungsmitteln, wobei wässrige Dispersionen besonders bevorzugte Beschichtungsmittel darstellen. Bevorzugt weisen die wässrigen Dispersionen einen Feststoffgehalt im Bereich von 10 bis 70 Gew.-%, besonders bevorzugt 20 bis 60 Gew.-% auf. Die dynamische Viskosität der Dispersion ist vom Feststoffgehalt und der Teilchengröße abhängig und kann einen weiten Bereich umfassen. So kann sie bei feinteiligen Dispersionen mit hohem Polymergehalt bei einigen bei mehr als 10.000 mPas liegen. Zweckmäßig ist meist eine dynamische Viskosität im Bereich von 10 bis 4000 mPas, bevorzugt 10 bis 1000 mPas und ganz besonders bevorzugt 10 bis 500 mPas, gemessen gemäß DIN EN ISO 2555 bei 25°C (Brookfield).

Weiterhin können die erfindungsgemäßen wässrigen Dispersionen auf bekannte Weise mit Additiven oder

weiteren Komponenten versehen werden, um die Eigenschaften des Beschichtungsmittels an spezifische Anforderungen anzupassen. Zu diesen Zusatzstoffen gehören insbesondere Fließverbesserer, Pigmente und Farbstoffe.

Vorzugsweise weisen die erfindungsgemäßen Beschichtungsmittel eine Mindest-Filmbildungs-Temperatur von höchstens 50°C, besonders bevorzugt höchstens 35°C und ganz besonders bevorzugt höchstens 25°C auf, die gemäß DIN ISO 2115 gemessen werden kann.

Zweckmäßig kann das Beschichtungsmittel, vorzugsweise eine wässrige Dispersion, eine Säurezahl im Bereich von 0 bis 100 mg KOH/g, bevorzugt 1 bis 40 mg KOH/g und ganz besonders bevorzugt im Bereich von 2 bis 10 mg KOH/g aufweisen. Die Säurezahl kann gemäß DIN EN ISO 2114 anhand einer Dispersion bestimmt werden, wobei sich der Wert auf den Feststoffgehalt bezieht.

Die Hydroxyzahl eines erfindungsgemäßen Beschichtungsmittels, insbesondere einer wässrigen Dispersion kann vorzugsweise im Bereich von 0 bis 400 mg KOH/g, besonders bevorzugt 1 bis 200 mg KOH/g und ganz besonders bevorzugt im Bereich von 3 bis 150 mg KOH/g liegen. Die Hydroxyzahl kann gemäß DIN EN ISO 4629 anhand einer Dispersion bestimmt werden, wobei sich der Wert auf den Feststoffgehalt bezieht.

Die erfindungsgemäßen Dispersionen können neben den Emulsionspolymeren auch weitere Bestandteile enthalten.

Die Vernetzung der erfindungsgemäßen Polymere bzw. Beschichtungsmittel kann durch Zugabe von Vernetzungsmitteln erfolgen: Hierfür geeignet sind nukleophile Verbindungen, die eine mehrfache Funktionalität aufweisen. Besonders geeignet sind unter anderem Diamine, beispielsweise 2,2'-(Ethylendioxy)diethylamin (Jeffamine® XTJ-504, CAS-Nr. 929-59-9), Harnstoff (CAS-Nr.57-13-6) und Harnstoff-Derivate, wie Ethylenharnstoff (auch Imidazolidinon genannt; CAS-Nr.120-93-4), Diphenylharnstoff (CAS-Nr.102-07-8), Diethylharnstoff (CAS-Nr.623-76-7) und Propylenharnstoff (auch Tetra-2-hydropyrimidinon genannt; CAS-Nr. 1852-17-1) und Dihydrazide, wie zum Beispiel Adipinsäuredihydrazid (ADH).

Besonders bevorzugte Vernetzungsmittel sind unter anderem in WO 94/25433, eingereicht am 25.04.1994 beim Europäischen Patentamt mit der Anmeldenummer PCT/EP94/01283 dargelegt, wobei auf diese Druckschrift zu Offenbarungszwecken verwiesen wird und die darin offenbarten Vernetzungsmittel in diese Anmeldung eingefügt werden.

Der Anteil an Vernetzungsmittel ist an sich nicht kritisch, wobei die Menge an Vernetzungsmittel vorzugsweise anhand des Polymers bemessen wird. Besonders bevorzugt liegt das molare Verhältnis von vernetzungsfähigen Gruppen, die im Polymer enthalten sind, zu den reaktiven Gruppen des Vernetzungsmittels im Bereich von 10:1 bis 1:10, besonders bevorzugt 4:1 bis 1:4 und ganz besonders bevorzugt im Bereich von 2:1 bis 1:2.

Die Polymere der vorliegenden Erfindung können insbesondere in Beschichtungsmitteln oder als Zusatzstoff eingesetzt werden. Hierzu gehören insbesondere Lacke, Imprägniermittel, Klebstoffe und/oder Grundierungen. Besonders-bevorzugt können die Beschichtungsmittel, insbesondere die wässrigen Dispersionen zur Beschichtung oder Imprägnierung von Leder und/oder Textilien, beispielsweise Geweben, Gewirken oder Vliesstoffen dienen. Weiterhin können die Dispersionen zur Beschichtung von Hölzern, Metallen und Kunststoffen eingesetzt werden. So zeigen insbesondere Lacke für Industriebeschichtungen und Bautenlacke eine ausgezeichnete Leistungsfähigkeit, wobei diese Lacke beispielsweise zur Beschichtung von Möbeln oder Bodenbelägen eingesetzt werden können.

Die aus den erfindungsgemäßen Beschichtungsmitteln erhältlichen Beschichtungen zeigen eine hohe Lösungsmittelbeständigkeit, wobei insbesondere nur geringe Anteile durch Lösungsmittel aus der Beschichtung gelöst werden. Bevorzugte Beschichtungen zeigen insbesondere gegenüber Methylisobutylketon (MIBK) eine hohe Beständigkeit. So beträgt der Gewichtsverlust nach einer Behandlung mit MIBK vorzugsweise höchstens 50 Gew.-%, bevorzugt höchstens 35 Gew.-%, insbesondere bevorzugt höchstens 20 Gew.-% und ganz besonders bevorzugt höchstens 15 Gew.-%, bezogen auf das Gewicht der eingesetzten Beschichtung. Die Aufnahme an MIBK beträgt vorzugsweise höchstens 1000 Gew.-%, besonders bevorzugt höchstens 800 Gew.-%, insbesondere bevorzugt höchstens 600 Gew.-% und ganz besonders bevorzugt höchstens 500 Gew.-%, bezogen auf das Gewicht der eingesetzten Beschichtung. Diese Werte werden bei einer Temperatur von ca. 25°C und einer Einwirkzeit von mindestens 4 Stunden gemessen, wobei eine vollständig getrocknete Beschichtung vermessen wird, die vernetzt wurde.

Die Beschichtungen, die aus den erfindungsgemäßen Beschichtungsmittel erhalten werden, zeigen eine hohe mechanische Beständigkeit. Bevorzugt beträgt die Pendelhärte mindestens 15 s, vorzugsweise mindestens 25 s, gemessen gemäß DIN ISO 1522.

Erfindungsgemäße Beschichtungen zeigen überraschend gute mechanische Eigenschaften. Von besonderem Interesse sind insbesondere Beschichtungen, die eine nominale Bruchdehnung von vorzugsweise mindestens 200 %, besonders bevorzugt mindestens 300 % aufweisen, gemessen gemäß DIN EN ISO 527 Teil 3.

Darüber hinaus sind weiterhin Beschichtungen bevorzugt, die eine Zugfestigkeit, gemessen gemäß DIN EN ISO 527 Teil 3, von mindestens 2 MPa, besonders bevorzugt mindestens 4 MPa zeigen.

Weiterhin stellt die vorliegende Erfindung überraschend Beschichtungen zur Verfügung, die bei einer Zugfestigkeit von mindestens 2 MPa, besonders bevorzugt mindestens

4 MPa, eine Bruchdehnung von mindestens 200 %, besonders bevorzugt mindestens 300 % aufweisen.

Weiterhin zeichnen sich bevorzugte Beschichtungen, die aus den erfindungsgemäßen Beschichtungsmitteln erhältlich sind, durch eine überraschend hohe Haftfestigkeit aus, die insbesondere gemäß dem Gitterschnitt-Versuch bestimmt werden kann. So kann insbesondere eine Klassifizierung von 0-1, besonders bevorzugt von 0 gemäß der Norm DIN EN ISO 2409 erzielt werden.

Nachfolgend soll die vorliegende Erfindung anhand von Beispielen und Vergleichsbeispielen näher erläutert werden, ohne dass hierdurch eine Beschränkung erfolgen soll.

**Herstellung von 4-Oxobutylmethacrylat**

In einem 100 ml Parr Autoklaven mit Druckkonstanthaltung aus einer konstant temperierten Bürette wurden 10 mmol Methacrylsäureallylester in 25 ml THF gegeben und bei einem Druck von 10 bar mit einem $CO/H_2$-Gasgemisch, welches ein molares Verhältnis von 1:1 aufwies, in Gegenwart von 0,1 mol.-% $Rh(acac)(CO)_2$ [acac = Acetylacetonat] und 0,2 mol.-% Xantphos, jeweils bezogen auf Methacrylsäureallylester, für eine Reaktionszeit von 20 Stunden bei einer Temperatur von 65°C umgesetzt. Es wurde eine Ausbeute von 90 % erzielt, wobei das Reaktionsgemisch 4-Oxobutylmethacrylat und
2-Methyl-3-oxopropylmethacrylat umfasste. Das Verhältnis von 4-Oxobutylmethacrylat zu 2-Methyl-3-oxopropylmethacrylat betrug 92:8.

**Herstellung einer Mischung von Methacryloyloxy-2-ethyl-fettsäureamiden**

In einem Vierhalsrundkolben, der mit einem Säbelrührer mit Rührhülse und Rührmotor, Stickstoffeinleitung, Sumpfthermometer und einer Destillationsbrücke ausgestattet war, wurden 206,3 g (0,70 mol) Fettsäuremethylestermischung, 42,8 g (0,70 mol) Ethanolamin und 0,27 g (0,26 %) LiOH vorgelegt. Die Fettsäuremethylestermischung umfasste 6 Gew.-% gesättigte C12 bis C16 Fettsäuremethylester, 2,5 Gew.-% gesättigte C17 bis C20 Fettsäuremethylester, 52 Gew.-% einfach ungesättigte C18 Fettsäuremethylester, 1,5 Gew.-% einfach ungesättigte C20 bis C24 Fettsäuremethylester, 36 Gew.-% mehrfach ungesättigte C18 Fettsäuremethylester, 2 Gew.-% mehrfach ungesättigte C20 bis C24 Fettsäuremethylester.

Das Reaktionsgemisch wurde auf 150°C aufgeheizt. Innerhalb von 2h wurden 19,5 ml Methanol abdestilliert. Das erhaltene Reaktionsprodukt enthielt 86,5% Fettsäureethanolamide. Das erhaltene Reaktionsgemisch wurde ohne Aufreinigung weiterverarbeitet.

Nach dem Abkühlen wurden 1919 g (19,2 mol) Methylmethacrylat, 3,1 g LiOH und ein Inhibitorgemisch bestehend aus 500 ppm Hydrochinonmonmethylether und 500 ppm Phenothiazin zugegeben.

Unter Rühren wurde die Reaktionsapparatur 10 Minuten mit Stickstoff gespült. Danach wurde das Reaktionsgemisch zum Sieden erhitzt. Das Methylmethacrylat/Methanol-Azeotrop wurde abgetrennt und anschließend die Kopftemperatur schrittweise auf 100°C angehoben. Nach Beendigung der Reaktion wurde das Reaktionsgemisch auf ca. 70°C abgekühlt und filtriert.

Am Rotationsverdampfer wurde überschüssiges Methylmethacrylat abgetrennt. Es konnten 370 g Produkt erhalten werden.

**Beispiel 1 (Herstellung einer Dispersion für Holz- und Metallbeschichtung)** BuA-co-MMA-ObMA-MAS = 46-51-2-1

Zunächst wurden in einem 1 l PE-Becherglas 110,4 g Butylacrylat (BA), 122,4 g Methylmethacrylat (MMA), 4,8 g Oxobutylmethacrylat (ObMA), 2,4 g Methacrylsäure (MAS), 0,72 g Ammoniumperoxodisulfat (APS), 7,2 g Disponil FES 32 (30 %ig) und 215,5 g Wasser mittels Ultra-Turrax 3 Minuten bei 4000 UpM emulgiert.

In einem 1 l Glasreaktor, der mit einem Wasserbad temperiert werden konnte und mit einem Blattrührer ausgestattet war, wurden 134 g Wasser und 0,18 g Disponil FES 32 (30 %ig) vorgelegt, auf 80°C erhitzt und mit 0,18 g Ammoniumperoxodisulfat (APS) gelöst in 10 g Wasser versetzt. 5 Minuten nach der APS-Zugabe wurde hierzu die zuvor hergestellte Emulsion innerhalb 240 Minuten (Intervall: 3 Minuten Zulauf, 4 Minuten Pause, 237 Minuten Restzulauf) zudosiert.

Nach Zulaufende wurde 1 Std. bei 80°C nachgerührt. Danach wurde auf Raumtemperatur abgekühlt und die Dispersion über VA-Siebgewebe mit 0,09 mm Maschenweite abfiltriert.

Die hergestellte Emulsion hatte einen Feststoffgehalt von 40 $\pm$ 1 %, einen pH-Wert von 2,2, eine Viskosität von 13 mPas und einen $r_{N5}$-Wert von 65 nm.

**Beispiel 2 (Herstellung einer Dispersion für Holz- und Metallbeschichtung)** BuA-co-MMA-ObMA-MAS = 45-50-4-1

Das Beispiel 1 wurde im Wesentlichen wiederholt, wobei jedoch eine Monomermischung als Zulauf verwendet wurde, die ca. 4 Gew.-% Oxobutylmethacrylat, bezogen auf das Gewicht der Monomere, umfasste. Zur Herstellung der

eingesetzten Monomermischung wurden in einem 1 l PE-Becherglas 105,96 g Butylacrylat (BA), 117,96 g Methylmethacrylat (MMA), 9,6 g Oxobutylmethacrylat, 2,4 g Methacrylsäure (MAS), 4,92 g Methacryloyloxy-2-ethyl-fettsäureamid-Mischung, 0,72 g Ammoniumperoxodisulfat (APS), 7,2 g Disponil FES 32 (30 %ig) und 208,6 g Wasser mittels Ultra-Turrax 3 Minuten bei 4000 UpM emulgiert.

Die hergestellte Emulsion hatte einen Feststoffgehalt von 40 ± 1 %, einen pH-Wert von 2,3, eine Viskosität von 13 mPas und einen $r_{N5}$-Wert von 65 nm.

**Beispiel 3 (Herstellung einer Dispersion für Holz- und Metallbeschichtung)** BuA-co-MMA-ObMA-MAS = 44-49-6-1

Das Beispiel 1 wurde im Wesentlichen wiederholt, wobei jedoch eine Monomermischung als Zulauf verwendet wurde, die ca. 6 Gew.-% Oxobutylmethacrylat, bezogen auf das Gewicht der Monomere, umfasste. Zur Herstellung der eingesetzten Monomermischung wurden in einem 1 l PE-Becherglas 105,96 g Butylacrylat (BA), 117,96 g Methylmethacrylat (MMA), 9,6 g Oxobutylmethacrylat, 2,4 g Methacrylsäure (MAS), 4,92 g Methacryloyloxy-2-ethyl-fettsäureamid-Mischung, 0,72 g Ammoniumperoxodisulfat (APS), 7,2 g Disponil FES 32 (30 %ig) und 208,6 g Wasser mittels Ultra-Turrax 3 Minuten bei 4000 UpM emulgiert.

Die hergestellte Emulsion hatte einen Feststoffgehalt von 40 ± 1 %, einen pH-Wert von 2,3, eine Viskosität von 14 mPas und einen $r_{N5}$-Wert von 68 nm.

**Beispiele 4 bis 6 (Vernetzung der in den Beispielen 1 bis 3 erhaltenen Dispersionen mit Adipinsäuredihydrazid)**

Die in den Beispielen 1 bis 3 erhaltenen Dispersionen wurden mit Adipinsäuredihydrazid (ADH) äquimolar vernetzt. Hierzu wurde eine 15 %ige ADH-Lösung unter Rühren zur Dispersion zugetropft, 2 h gerührt und ein Film bei Raumtemperatur ausgetrocknet.

Die Eigenschaften des so erhaltenen Beschichtungsmittels wurden anhand von unterschiedlichen Verfahren untersucht. Hierzu wurden an getrockneten Filmen Versuche zur Lösungsmittelbeständigkeit, der Wasseraufnahme und der Härte durchgeführt.

Die Bestimmung der Lösungsmittelbeständigkeit erfolgte unter Verwendung von Methylisobutylketon (MIBK), wobei eine Probe (A) mit MIBK bei Raumtemperatur 4 Stunden gequollen wurde. Anschließend wurde die Probe aus dem Lösungsmittel genommen, überschüssiges Lösungsmittel entfernt und das Gewicht bestimmt. Anschließend wurde die Probe 1 Stunde bei ca. 140°C getrocknet (B). Aus der Gewichtsdifferenz zwischen A und B errechnet sich der durch das Lösungsmittel entfernte Anteil der Probe. Die Quellung wurde bezogen auf das Gewicht der von löslichen Anteilen befreiten Probe B berechnet und wird im weiteren Text als "wahre Quellung" bezeichnet.

Ferner wurde ein Möbeltest analog DIN 68861-1 durchgeführt. Bewertungsskala 1 - 5, dabei bedeutet 5 = keine sichtbare Veränderung und 1 = Prüffläche stark verändert oder zerstört.

Die Bestimmung der Haftung wurde mit dem Gitterschnitt-Test gemäß DIN ISO 2409 durchgeführt.

Die erhaltenen Ergebnisse sind in Tabelle 1 aufgeführt. Das Beispiel 4 wurde unter Verwendung einer Dispersion gemäß Beispiel 1, Beispiel 5 unter Verwendung einer Dispersion gemäß Beispiel 2 und Beispiel 6 unter Verwendung einer Dispersion gemäß Beispiel 3 erhalten.

**Vergleichsbeispiel 1**

BuA-co-MMA-AAEMA-MAS = 46-51-2-1

Zum Vergleich wurde anstelle Oxobutylmethacrylat das kommerziell erhältliche Acetoacetoxyethylmethacrylat (AAEMA) in Dispersionen eingebaut.

Dementsprechend wurde das Beispiel 1 im Wesentlichen wiederholt, wobei jedoch eine Monomermischung als Zulauf verwendet wurde, die ca. 2 Gew.-% Acetoacetoxyethylmethacrylat anstatt Oxobutylmethacrylat umfasste, bezogen auf das Gewicht der Monomere. Zur Herstellung der eingesetzten Monomermischung wurden in einem 1 l PE-Becherglas 110,4 g Butylacrylat (BA), 122,4 g Methylmethacrylat (MMA), 4,8 g Acetoacetoxyethylmethacrylat (AAEMA), 2,4 g Methacrylsäure (MAS), 0,72 g Ammoniumperoxodisulfat (APS), 7,2 g Disponil FES 32 (30 %ig) und 215,5 g Wasser mittels Ultra-Turrax 3 Minuten bei 4000 UpM emulgiert.

Die hergestellte Emulsion hatte einen Feststoffgehalt von 40 ± 1 %, einen pH-Wert von 2,4, eine Viskosität von 12 mPas und einen $r_{N5}$-Wert von 65 nm.

**Vergleichsbeispiel 2**

BuA-co-MMA-AAEMA-MAS = 45-50-4-1

Das Vergleichsbeispiel 1 wurde im Wesentlichen wiederholt, wobei jedoch eine Monomermischung als Zulauf verwendet wurde, die ca. 4 Gew.-% Acetoacetoxyethylmethacrylat, bezogen auf das Gewicht der Monomere, umfasste. Zur Herstellung der eingesetzten Monomermischung wurden in einem 1 l PE-Becherglas 108 g Butylacrylat (BA), 120 g Methylmethacrylat (MMA), 9,6 g Acetoacetoxyethylmethacrylat (AAEMA), 2,4 g Methacrylsäure (MAS), 0,72 g Ammoniumperoxodisulfat (APS), 7,2 g Disponil FES 32 (30 %ig) und 215,5 g Wasser mittels Ultra-Turrax 3 Minuten bei 4000 UpM emulgiert.

Die hergestellte Emulsion hatte einen Feststoffgehalt von 40 ± 1 %, einen pH-Wert von 2,4, eine Viskosität von 14 mPas und einen $r_{N5}$-Wert von 62 nm.

**Vergleichsbeispiel 3**

BuA-co-MMA-AAEMA-MAS = 44-49-6-1

Das Vergleichsbeispiel 1 wurde im Wesentlichen wiederholt, wobei jedoch eine Monomermischung als Zulauf verwendet wurde, die ca. 6 Gew.-% Acetoacetoxyethylmethacrylat, bezogen auf das Gewicht der Monomere, umfasste. Zur Herstellung der eingesetzten Monomermischung wurden in einem 1 l PE-Becherglas 105,6 g Butylacrylat (BA), 117,6 g Methylmethacrylat (MMA), 14,4 g Acetoacetoxyethylmethacrylat (AAEMA), 2,4 g Methacrylsäure (MAS), 0,72 g Ammoniumperoxodisulfat (APS), 7,2 g Disponil FES 32 (30 %ig) und 215,5 g Wasser mittels Ultra-Turrax 3 Minuten bei 4000 UpM emulgiert.

Die hergestellte Emulsion hatte einen Feststoffgehalt von 40 ± 1 %, einen pH-Wert von 2,4, eine Viskosität von 13 mPas und einen $r_{N5}$-Wert von 61 nm.

**Vergleichsbeispiele 4 bis 6 (Vernetzung der in den Vergleichsbeispielen 1 bis 3 erhaltenen Dispersionen mit Dihydrazid)**

Die in den Vergleichsbeispielen 1 bis 3 erhaltenen Dispersionen wurden mit Adipinsäuredihydrazid (ADH) äquimolar vernetzt. Hierzu wurde eine 15 %ige ADH-Lösung unter Rühren zur Dispersion zugetropft, gerührt und ein Film bei Raumtemperatur aufgetrocknet.

Die Eigenschaften des so erhaltenen Beschichtungsmittels wurden anhand von unterschiedlichen Verfahren untersucht. Hierzu wurden an getrockneten Filmen Versuche zur Lösungsmittelbeständigkeit, der Haftung und der Härte durchgeführt, wie dies zuvor im Zusammenhang mit den Beispielen 4 bis 6 dargelegt wurde.

Das Vergleichsbeispiel 4 wurde unter Verwendung einer Dispersion gemäß Vergleichsbeispiel 1, Vergleichsbeispiel 5 unter Verwendung einer Dispersion gemäß Vergleichsbeispiel 2 und Vergleichsbeispiel 6 unter Verwendung einer Dispersion gemäß Vergleichsbeispiel 3 erhalten.

Die erhaltenen Ergebnisse sind in Tabelle 1 aufgeführt.

**Tabelle 1:** Ergebnisse der Eigenschaftsuntersuchungen

| | Beispiel 4 | Beispiel 5 | Beispiel 6 |
|---|---|---|---|
| Pendelhärte [s] nach 1 Woche | 68,6 | 93,8 | 120,4 |
| wahre Quellung in MIBK [%] | 981 | 715 | 480 |
| Gewichtsverlust in MIBK [%] | 16,7 | 14,0 | 5,9 |
| Gitterschnitt nach ISO 2409 ohne/mit Tesa | 0/0 | 0/0 | 0/0 |
| Möbeltest Ethanol nach 1h/16h | 5/5 | 5/5 | 5/5 |
| Möbeltest HOAc nach 1h/16h | 5/5 | 5/5 | 5/5 |
| Möbeltest NH3 nach 1h/16h | 4/4 | 4/4 | 5/5 |
| Möbeltest Nivea nach 1h/16h | 5/5 | 5/5 | 5/5 |

**Tabelle 1:** Ergebnisse der Eigenschaftsuntersuchungen

| | Vergleichs-beispiel 4 | Vergleichs-beispiel 5 | Vergleichs-beispiel 6 |
|---|---|---|---|
| Pendelhärte [s] nach 1 Woche | 60,2 | 77,0 | 86,8 |
| wahre Quellung in MIBK [%] | 1014 | 955 | 607 |
| Gewichtsverlust in MIBK [%] | 20,8 | 17,4 | 8,3 |
| Gitterschnitt nach ISO 2409 | 0/0 | 0/0 | 0/0 |
| Möbeltest Ethanol nach 1h/16h | 5/5 | 5/5 | 5/5 |
| Möbeltest HOAc nach 1h/16h | 5/5 | 5/5 | 5/5 |
| Möbeltest NH3 nach 1h/16h | 4/3 | 4/4 | 4/4 |
| Möbeltest Nivea nach 1h/16h | 5/5 | 5/5 | 5/5 |

Die gemäß den Beispielen 4 bis 6 erhaltenen Dispersionen zeigen ein hervorragendes Eigensehaftsspektrum hinsichtlich eines Beschichtungsmittels für Holz und Metall.

**Beispiel 7**

Das Beispiel 2 wurde im Wesentlichen wiederholt, wobei jedoch eine Monomermischung als Zulauf verwendet wurde, die 1,7 Gew.-% Methacrylamid, bezogen auf das Gewicht der Monomere, umfasste. Zur Herstellung der eingesetzten Monomermischung wurden in einem 1 l PE-Becherglas 105,96 g Butylacrylat (BA), 117,96 g Methylmethacrylat (MMA), 9,6 g Oxobutylmethacrylat, 2,4 g Methacrylsäure (MAS), 4,08 g Methacrylamid (MAA), 0,72 g Ammoniumperoxodisulfat (APS), 7,2 g Disponil FES 32 (30 %ig) und 208,6 g Wasser mittels Ultra-Turrax 3 Minuten bei 4000 UpM emulgiert.

Die hergestellte Emulsion hatte einen Feststoffgehalt von 40 $\pm$ 1 %, eine Viskosität von 12 mPas und einen $r_{N5}$-Wert von 66 nm.

**Beispiel 8**

Das Beispiel 2 wurde im Wesentlichen wiederholt, wobei jedoch eine Monomermischung als Zulauf verwendet wurde, die ca. 2 Gew.-% Methacryloyloxy-2-ethylfettsäureamid-Mischung, bezogen auf das Gewicht der Monomere, umfasste. Zur Herstellung der eingesetzten Monomermischung wurden in einem 1 l PE-Becherglas 105,96 g Butylacrylat (BA), 117,96 g Methylmethacrylat (MMA), 9,6 g Oxobutylmethacrylat, 2,4 g Methacrylsäure (MAS), 4,92 g Methacryloyloxy-2-ethyl-fettsäureamid-Mischung, 0,72 g Ammoniumperoxodisulfat (APS), 7,2 g Disponil FES 32 (30 %ig) und 208,6 g Wasser mittels Ultra-Turrax 3 Minuten bei 4000 UpM emulgiert.

Die hergestellte Emulsion hatte einen Feststoffgehalt von 39 $\pm$ 1 %, eine Viskosität von 11 mPas und einen $r_{N5}$-Wert von 65 nm.

**Beispiele 9 bis 11**

Die Emulsionen der Beispiele 2, 7 und 9 wurden einer Selbstvernetzung unterzogen. Hierzu wurde der pH-Wert aller Dispersionen auf einen Wert von 5,8 eingestellt. Aus den Dispersionen wurden Beschichtungen hergestellt, die deren Quellung in (MIBK) untersucht wurden. Hierbei wurde ein Film bei Raumtemperatur hergestellt und 24 Stunden bei dieser Temperatur getrocknet.

Die Bestimmung der Lösungsmittelbeständigkeit erfolgte unter Verwendung von Methylisobutylketon (MIBK), wobei eine Probe mit MIBK bei Raumtemperatur 4 Stunden gequollen wurde. Anschließend wurde die Probe aus dem Lösungsmittel genommen und überschüssiges Lösungsmittel entfernt. Aus dem Gewicht der gequollenen Probe wurde die Lösungsmittelaufnahme berechnet. Anschließend wurde die Probe 1 Stunde bei ca. 140°C getrocknet. Aus dem Gewichtsverlust errechnet sich der durch das Lösungsmittel entfernte Anteil der Probe.

Die erhaltenen Ergebnisse sind in Tabelle 2 dargelegt. Das Beispiel 9 wurde unter Verwendung einer Dispersion gemäß Beispiel 2, Beispiel 10 unter Verwendung einer Dispersion gemäß Beispiel 7 und Beispiel 11 unter Verwendung einer Dispersion gemäß Beispiel 8 erhalten.

Tabelle 2-

| | Lösungsmittelaufnahme [%] | Gewichtsverlust nach Rücktrocknung [%] | wahre Quellung [%] |
|---|---|---|---|
| Beispiel 9 | 992 | 24,1 | 1307 |
| Beispiel 10 | 565 | 11,4 | 637 |
| Beispiel 11 | 814 | 17,9 | 991 |

**Beispiel 12 (Herstellung einer Dispersion für Textilanwendungen auf Basis: BA-co-MMA-Oxobutylmethacrylat-HEMA)**

Zunächst wurden in einem 2 l PE-Becherglas 210,4 g Butylacrylat (BA), 167,6 g Methylmethacrylat (MMA), 12 g Oxobutylmethacrylat (ObMA), 10 g 2-Hydroxyethylmethacrylat (HEMA), 12 g 4,4'-Azobis-(4-cyanovaleriansäure), 12 g Disponil FES 32 (30 %ig) und 345,68 g Wasser mittels Ultra-Turrax 3 Minuten bei 4000 UpM emulgiert.

In einem 2 l Glasreaktor, der mit einem Wasserbad temperiert werden konnte und mit einem Blattrührer ausgestattet war, wurden 240 g Wasser und 0,3 g Disponil FES 32 (30 %ig) vorgelegt, auf 80°C erhitzt und mit 3 g 4,4'-Azobis-(4-cyanovaleriansäure) versetzt. 5 Minuten nach der Initiator-Zugabe wurde hierzu die zuvor hergestellte Emulsion innerhalb 240 Minuten (Intervall: 3 Minuten Zulauf, 4 Minuten Pause, 237 Minuten Restzulauf) zudosiert. Zur Dosierung der Emulsion aus einem Zulaufgefäß wurde eine Lewa-Kolbendosierpumpe, Typ HK mit 5 mm Kolben eingestellt auf eine Förderleistung von 0,193 kg/h, verwendet.

Nach Zulaufende wurde 1 Std. bei 80°C nachgerührt. Danach wurde auf Raumtemperatur abgekühlt und die Dispersion über VA-Siebgewebe mit 0,09 mm Maschenweite abfiltriert.

Die hergestellte Emulsion hatte einen Feststoffgehalt von 40 $\pm$ 1 %, einen pH-Wert von 6,4, eine Viskosität von 11 mPas und einen $r_{N5}$-Wert von 107 nm.
Anschließend erfolgte eine pH-Einstellung mit Phosphorsäure auf pH ~ 2,0.

**Beispiel 13 (Herstellung einer Dispersion für Textilanwendungen auf Basis: BA-co-MMA-Oxobutylmethacrylat-MAA)**

Das Beispiel 12 wurde im Wesentlichen wiederholt. Allerdings wurde als Zulauf eine Monomermischung eingesetzt, die Methacrylamid umfasst. Zur Herstellung dieser Monomermischung wurden in einem 2 l PE-Becherglas 212 g Butylacrylat (BA), 169,2 g Methylmethacrylat (MMA), 12 g Oxobutylmethacrylat (ObMA), 6,8 g Methacrylamid (MAA), 12 g 4,4'-Azobis-(4-cyanovaleriansäure), 12 g Disponil FES 32 (30 %ig) und 345,68 g Wasser mittels Ultra-Turrax 3 Minuten bei 4000 UpM emulgiert.

Die hergestellte Emulsion hatte einen Feststoffgehalt von 40 $\pm$ 1 %, einen pH-Wert von 6,3, eine Viskosität von 12 mPas und einen $r_{N5}$-Wert von 93 nm. Anschließend erfolgte eine pH-Einstellung mit Phosphorsäure auf pH ~ 2,0.

Aus den in Beispielen 12 und 13 dargelegten Dispersionen wurden Beschichtungen hergestellt, deren mechanischen Eigenschaften sowie Quellung in einem Lösemittel (MIBK) untersucht wurden. Hierbei wurde ein Film bei Raumtemperatur hergestellt und 24 Stunden bei dieser Temperatur getrocknet. Ein weiterer Film wurde bei Raumtemperatur hergestellt, jedoch zusätzlich für 30 Minuten auf 140°C erhitzt. Die Ergebnisse sind in Tabellen 2 bis 5 zusammengefasst.

**Tabelle 2: Quellversuche in MIBK (4 Std.); Filmherstellung bei RT**

| | Lösungsmittelaufnahme [%] | Gewichtsverlust nach Rücktrocknung [%] | wahre Quellung [%] |
|---|---|---|---|
| Beispiel 12 | 449 | 13,1 | 532 |
| Beispiel 13 | 355 | 9,1 | 400 |

**Tabelle 3: Quellversuche in MIBK (4 Std.); Film 30 Min. bei 140°C getempert**

| | Lösungsmittelaufnahme [%] | Gewichtsverlust nach Rücktrocknung [%] | wahre Quellung [%] |
|---|---|---|---|
| Beispiel 12 | 350 | 5,9 | 378 |
| Beispiel 13 | 287 | 4,8 | 307 |

**Tabelle 4: Mechanische Eigenschaften der Filme; Filmherstellung bei RT**

| | (nom.) Bruchdehnung [%] | Zugfestigkeit [MPa] |
|---|---|---|
| Beispiel 12 | 410,5 | 8,3 |
| Beispiel 13 | 327,4 | 8 |

**Tabelle 5: Mechanische Eigenschaften der Filme; Film 30 Min. bei 140°C getempert**

| | (nom.) Bruchdehnung [%] | Zugfestigkeit [MPa] |
|---|---|---|
| Beispiel 12 | 350,1 | 5,5 |
| Beispiel 13 | 329,4 | 11,1 |

## Patentansprüche

**1.** Monomermischung umfassend
mindestens 30 Gew.-% eines oder mehrerer Alkyl(meth)acrylate mit 1 bis 10 Kohlenstoffatomen im Alkylrest, deren Alkylrest keine Doppelbindungen oder Heteroatome aufweist, und
0,1 bis 40 Gew.-% eines oder mehrerer (Meth)acrylate mit mindestens einer Aldehydgruppe im Alkylrest, **dadurch gekennzeichnet, dass** das (Meth)acrylat mit mindestens einer Aldehydgruppe im Alkylrest am Kohlenstoffatom in alpha-Stellung zur Aldehydgruppe ein Wasserstoffatom aufweist.

**2.** Monomermischung gemäß Anspruch 1, **dadurch gekennzeichnet, dass** das (Meth)acrylat mit mindestens einer Aldehydgruppe im Alkylrest 3 bis 9 Kohlenstoffatome im Alkylrest aufweist, vorzugsweise ausgewählt ist aus 4-Oxobutyl(meth)acrylat und 2-Methyl-3-oxopropyl(meth)acrylat.

**3.** Monomermischung gemäß Anspruch 1 oder 2, **dadurch gekennzeichnet, dass** die Monomermischung mindestens ein (Meth)acrylmonomer mit einer Amino-oder Amidgruppe aufweist, vorzugsweise ein Monomer mit einer primären Amidgruppe ($-CO-NH_2$) oder einer sekundären Amidgruppe (-CO-NHR) ist.

**4.** Monomermischung gemäß Anspruch 3, **dadurch gekennzeichnet, dass** das (Meth)acrylmonomer ein Monomer mit einer primären oder tertiären Aminogruppe ist.

**5.** Monomermischung gemäß Anspruch 3 oder 4, **dadurch gekennzeichnet, dass** die Monomermischung 0,1 bis 10 Gew.-% Monomer mit einer Amino- oder Amidgruppe aufweist.

**6.** Monomermischung gemäß mindestens einem der vorhergehenden Ansprüche, **dadurch gekennzeichnet, dass** die Monomermischung mindestens ein Polyalkylenglykolmono(meth)acrylat aufweist.

**7.** Monomermischung gemäß Anspruch 6, **dadurch gekennzeichnet, dass** das Polyalkylenglykolmono(meth)acrylat

ein Gewichtsmittel des Molekulargewichts im Bereich von 350 bis 20000 g/mol aufweist.

8. Monomermischung gemäß Anspruch 6 oder 7, **dadurch gekennzeichnet, dass** das Polyalkylenglykolmono(meth)acrylat ein Methoxypolyethylenglykolmonomethacrylat ist.

9. Monomermischung gemäß mindestens einem der vorhergehenden Ansprüche, **dadurch gekennzeichnet, dass** die Monomermischung mindestens 30 Gew.-% Alkylacrylat mit 1 bis 10 Kohlenstoffatomen, dessen Alkylrest keine Doppelbindungen oder Heteroatome aufweist, umfasst.

10. Monomermischung gemäß Anspruch 9, **dadurch gekennzeichnet, dass** das Alkylacrylat mit 1 bis 10 Kohlenstoffatomen im Alkylrest ausgewählt aus Ethylacrylat, Butylacrylat und/oder Ethylhexylacrylat.

11. Monomermischung gemäß mindestens einem der vorhergehenden Ansprüche, **dadurch gekennzeichnet, dass** die Monomermischung mindestens 30 Gew.-% Alkylmethacrylat mit 1 bis 10 Kohlenstoffatomen, dessen Alkylrest keine Doppelbindungen oder Heteroatome aufweist, umfasst.

12. Monomermischung gemäß Anspruch 11, **dadurch gekennzeichnet, dass** das Alkylmethacrylat ausgewählt ist aus Methylmethacrylat und Cycloalkylmethacrylaten.

13. Monomermischung gemäß mindestens einem der vorhergehenden Ansprüche, **dadurch gekennzeichnet, dass** die Monomermischung mindestens ein Alkylacrylat mit 1 bis 10 Kohlenstoffatomen im Alkylrest, dessen Alkylrest keine Doppelbindungen oder Heteroatome aufweist, und mindestens ein Alkylmethacrylat mit 1 bis 10 Kohlenstoffatomen im Alkylrest, dessen Alkylrest keine Doppelbindungen oder Heteroatome aufweist, umfasst, wobei das Gewichtsverhältnis von Alkylacrylat mit 1 bis 10 Kohlenstoffatomen im Alkylrest zu Alkylmethacrylat mit 1 bis 10 Kohlenstoffatomen im Alkylrest im Bereich von 1:1 bis 50:1 liegt.

14. Monomermischung gemäß mindestens einem der vorhergehenden Ansprüche 1 bis 12, **dadurch gekennzeichnet, dass** die Monomermischung mindestens ein Alkylacrylat mit 1 bis 10 Kohlenstoffatomen im Alkylrest, dessen Alkylrest keine Doppelbindungen oder Heteroatome aufweist, und mindestens ein Alkylmethacrylat mit 1 bis 10 Kohlenstoffatomen im Alkylrest, dessen Alkylrest keine Doppelbindungen oder Heteroatome aufweist, umfasst, wobei das Gewichtsverhältnis von Alkylacrylat mit 1 bis 10 Kohlenstoffatomen im Alkylrest zu Alkylmethacrylat mit 1 bis 10 Kohlenstoffatomen im Alkylrest im Bereich von 1:50 bis 1:1 liegt.

15. Polymer umfassend Wiederholungseinheiten, abgeleitet von einer Monomermischung gemäß mindestens einem der Ansprüche 1 bis 14.

16. Polymer gemäß Anspruch 15, **dadurch gekennzeichnet, dass** das Polymer ein Emulsionspolymer ist.

17. Polymer gemäß Anspruch 15, **dadurch gekennzeichnet, dass** das Polymer ein Kern-Schale-Polymer ist.

18. Polymer gemäß Anspruch 17, **dadurch gekennzeichnet, dass** der Kern des Kern-Schale-Polymers mindestens 50 bis 100 Gew.-% (Meth)acrylate umfasst.

19. Beschichtungsmittel, **dadurch gekennzeichnet, dass** das Beschichtungsmittel ein Polymer gemäß mindestens einem der Ansprüche 15 bis 18 umfasst.

20. Verfahren zur Herstellung einer Beschichtung, **dadurch gekennzeichnet, dass** ein Beschichtungsmittel gemäß Anspruch 19 auf ein Substrat aufgebracht und gehärtet wird.

21. Verfahren gemäß Anspruch 20, **dadurch gekennzeichnet, dass** das Beschichtungsmittel auf Holz, Metall, Kunststoff, Textilien, Vliesstoffen und/oder Leder aufgebracht wird.

22. Verfahren gemäß mindestens einem der Ansprüche 20 bis 21, **dadurch gekennzeichnet, dass** das Polymer mit einem Diamin und/oder einem Dihydrazid umgesetzt wird.

23. Verfahren gemäß mindestens einem der Ansprüche 20 bis 22, **dadurch gekennzeichnet, dass** das Polymer durch Temperung bei einer Temperatur über 40°C einer Selbstvernetzung unterzogen wird.

**24.** Beschichteter Gegenstand, erhältlich durch ein Verfahren gemäß mindestens einem der Ansprüche 20 bis 23.

## Claims

**1.** Monomer mixture comprising
at least 30% by weight of one or more alkyl (meth)acrylates having 1 to 10 carbon atoms in the alkyl radical, whose alkyl radical contains no double bonds or heteroatoms, and
0.1% to 40% by weight of one or more (meth)-acrylates having at least one aldehyde group in the alkyl radical, **characterized in that** the (meth)acrylate having at least one aldehyde group in the alkyl radical contains a hydrogen atom on the carbon atom in alpha-position to the aldehyde group.

**2.** Monomer mixture according to Claim 1, **characterized in that** the (meth)acrylate having at least one aldehyde group in the alkyl radical contains 3 to 9 carbon atoms in the alkyl radical and is preferably selected from 4-oxobutyl (meth)acrylate and 2-methyl-3-oxopropyl (meth)-acrylate.

**3.** Monomer mixture according to Claim 1 or 2, **characterized in that** the monomer mixture comprises at least one (meth)acrylic monomer having an amino group or amide group, preferably a monomer having a primary amide group ($-CO-NH_2$) or a secondary amide group (-CO-NHR).

**4.** Monomer mixture according to Claim 3, **characterized in that** the (meth)acrylic monomer is a monomer having a primary or tertiary amino group.

**5.** Monomer mixture according to Claim 3 or 4, **characterized in that** the monomer mixture contains 0.1% to 10% by weight of monomer having an amino group or amide group.

**6.** Monomer mixture according to at least one of the preceding claims, **characterized in that** the monomer mixture comprises at least one polyalkylene glycol mono(meth)acrylate.

**7.** Monomer mixture according to Claim 6, **characterized in that** the polyalkylene glycol mono(meth)acrylate has a weight-average molecular weight in the range from 350 to 20 000 g/mol.

**8.** Monomer mixture according to Claim 6 or 7, **characterized in that** the polyalkylene glycol mono(meth)acrylate is a methoxypolyethylene glycol monomethacrylate.

**9.** Monomer mixture according to at least one of the preceding claims, **characterized in that** the monomer mixture comprises at least 30% by weight of alkyl acrylate having 1 to 10 carbon atoms, whose alkyl radical contains no double bonds or heteroatoms.

**10.** Monomer mixture according to Claim 9, **characterized in that** the alkyl acrylate having 1 to 10 carbon atoms in the alkyl radical is selected from ethyl acrylate, butyl acrylate and/or ethylhexyl acrylate.

**11.** Monomer mixture according to at least one of the preceding claims, **characterized in that** the monomer mixture comprises at least 30% by weight of alkyl methacrylate having 1 to 10 carbon atoms, whose alkyl radical contains no double bonds or heteroatoms.

**12.** Monomer mixture according to Claim 11, **characterized in that** the alkyl methacrylate is selected from methyl methacrylate and cycloalkyl methacrylates.

**13.** Monomer mixture according to at least one of the preceding claims, **characterized in that** the monomer mixture comprises at least one alkyl acrylate having 1 to 10 carbon atoms in the alkyl radical, whose alkyl radical contains no double bonds or heteroatoms, and at least one alkyl methacrylate having 1 to 10 carbon atoms in the alkyl radical, whose alkyl radical contains no double bonds or heteroatoms, the weight ratio of alkyl acrylate having 1 to 10 carbon atoms in the alkyl radical to alkyl methacrylate having 1 to 10 carbon atoms in the alkyl radical being situated in the range from 1:1 to 50:1.

**14.** Monomer mixture according to at least one of the preceding Claims 1 to 12, **characterized in that** the monomer mixture comprises at least one alkyl acrylate having 1 to 10 carbon atoms in the alkyl radical, whose alkyl radical

contains no double bonds or heteroatoms, and at least one alkyl methacrylate having 1 to 10 carbon atoms in the alkyl radical, whose alkyl radical contains no double bonds or heteroatoms, the weight ratio of alkyl acrylate having 1 to 10 carbon atoms in the alkyl radical to alkyl methacrylate having 1 to 10 carbon atoms in the alkyl radical being situated in the range from 1:50 to 1:1.

**15.** Polymer comprising repeating units derived from a monomer mixture according to at least one of Claims 1 to 14.

**16.** Polymer according to Claim 15, **characterized in that** the polymer is an emulsion polymer.

**17.** Polymer according to Claim 15, **characterized in that** the polymer is a core-shell polymer.

**18.** Polymer according to Claim 17, **characterized in that** the core of the core-shell polymer comprises at least 50% to 100% by weight of (meth)acrylates.

**19.** Coating material **characterized in that** the coating material comprises a polymer according to at least one of Claims 15 to 18.

**20.** Method of producing a coating, **characterized in that** a coating material according to Claim 19 is applied to a substrate and cured.

**21.** Method according to Claim 20, **characterized in that** the coating material is applied to wood, metal, plastic, textiles, nonwovens and/or leather.

**22.** Method according to at least one of Claims 20 to 21, **characterized in that** the polymer is reacted with a diamine and/or a dihydrazide.

**23.** Method according to at least one of Claims 20 to 22, **characterized in that** the polymer is subjected to self-crosslinking by heat treatment at a temperature above 40°C.

**24.** Coated article obtainable by a method according to at least one of Claims 20 to 23.

## Revendications

**1.** Mélange de monomères, comprenant
au moins 30% en poids d'un ou de plusieurs (méth)acrylates d'alkyle comprenant 1 à 10 atomes de carbone dans le radical alkyle, dont le radical alkyle ne présente pas de doubles liaisons ni d'hétéroatomes, et
0,1 à 40% en poids d'un ou de plusieurs (méth)acrylates comprenant au moins un groupe aldéhyde dans le radical alkyle, **caractérisé en ce que** le (méth)acrylate comprenant au moins un groupe aldéhyde dans le radical alkyle présente un atome d'hydrogène sur l'atome de carbone en position alpha par rapport au groupe aldéhyde.

**2.** Mélange de monomères selon la revendication 1, **caractérisé en ce que** le (méth)acrylate comprenant au moins un groupe aldéhyde dans le radical alkyle présente 3 à 9 atomes de carbone dans le radical alkyle, de préférence choisi parmi le (méth)acrylate de 4-oxobutyle et le (méth)acrylate de 2-méthyl-3-oxopropyle.

**3.** Mélange de monomères selon la revendication 1 ou 2, **caractérisé en ce que** le mélange de monomères présente au moins un monomère de type (méth)acryle présentant un groupe amino ou amide, de préférence un monomère présentant un groupe amide primaire ($-CO-NH_2$) ou un groupe amide secondaire (-CO-NHR).

**4.** Mélange de monomères selon la revendication 3, **caractérisé en ce que** le monomère de (méth)acryle est un monomère présentant un groupe amino primaire ou tertiaire.

**5.** Mélange de monomères selon la revendication 3 ou 4, **caractérisé en ce que** le mélange de monomères présente 0,1 à 10% en poids de monomère présentant un groupe amino ou amide.

**6.** Mélange de monomères selon au moins l'une quelconque des revendications précédentes, **caractérisé en ce que** le mélange de monomères présente au moins un mono(méth)acrylate de polyalkylèneglycol.

**7.** Mélange de monomères selon la revendication 6, **caractérisé en ce que** le mono(méth)acrylate de polyalkylèneglycol présente un poids moléculaire pondéral moyen dans la plage de 350 à 20000 g/mole.

**8.** Mélange de monomères selon la revendication 6 ou 7, **caractérisé en ce que** le mono(méth)acrylate de polyalkylèneglycol est un monométhacrylate de méthoxypolyéthylèneglycol.

**9.** Mélange de monomères selon au moins l'une quelconque des revendications précédentes, **caractérisé en ce que** le mélange de monomères comprend au moins 30% en poids d'acrylate d'alkyle comprenant 1 à 10 atomes de carbone, dont le radical alkyle ne présente pas de doubles liaisons ni d'hétéroatomes.

**10.** Mélange de monomères selon la revendication 9, **caractérisé en ce que** l'acrylate d'alkyle comprenant 1 à 10 atomes de carbone dans le radical alkyle est choisi parmi l'acrylate d'éthyle, l'acrylate de butyle et/ou l'acrylate d'éthylhexyle.

**11.** Mélange de monomères selon au moins l'une quelconque des revendications précédentes, **caractérisé en ce que** le mélange de monomères comprend au moins 30% en poids de méthacrylate d'alkyle comprenant 1 à 10 atomes de carbone, dont le radical alkyle ne présente pas de doubles liaisons ni d'hétéroatomes.

**12.** Mélange de monomères selon la revendication 11, **caractérisé en ce que** le méthacrylate d'alkyle est choisi parmi le méthacrylate de méthyle et les méthacrylates de cycloalkyle.

**13.** Mélange de monomères selon au moins l'une quelconque des revendications précédentes, **caractérisé en ce que** le mélange de monomères présente au moins un acrylate d'alkyle comprenant 1 à 10 atomes de carbone dans le radical alkyle, dont le radical alkyle ne présente pas de doubles liaisons ni d'hétéroatomes, et au moins un méthacrylate d'alkyle comprenant 1 à 10 atomes de carbone dans le radical alkyle, dont le radical alkyle ne présente pas de doubles liaisons ni d'hétéroatomes, le rapport pondéral d'acrylate d'alkyle comprenant 1 à 10 atomes de carbone dans le radical alkyle à méthacrylate d'alkyle comprenant 1 à 10 atomes de carbone dans le radical alkyle se situant dans la plage de 1:1 à 50:1.

**14.** Mélange de monomères selon au moins l'une quelconque des revendications précédentes 1 à 12, **caractérisé en ce que** le mélange de monomères présente au moins un acrylate d'alkyle comprenant 1 à 10 atomes de carbone dans le radical alkyle, dont le radical alkyle ne présente pas de doubles liaisons ni d'hétéroatomes, et au moins un méthacrylate d'alkyle comprenant 1 à 10 atomes de carbone dans le radical alkyle, dont le radical alkyle ne présente pas de doubles liaisons ni d'hétéroatomes, le rapport pondéral d'acrylate d'alkyle comprenant 1 à 10 atomes de carbone dans le radical alkyle à méthacrylate d'alkyle comprenant 1 à 10 atomes de carbone dans le radical alkyle se situant dans la plage de 1:50 à 1:1.

**15.** Polymère comprenant des unités récurrentes dérivées d'un mélange de monomères selon au moins l'une quelconque des revendications 1 à 14.

**16.** Polymère selon la revendication 15, **caractérisé en ce que** le polymère est un polymère en émulsion.

**17.** Polymère selon la revendication 15, **caractérisé en ce que** le polymère est un polymère à noyau-coquille.

**18.** Polymère selon la revendication 17, **caractérisé en ce que** le noyau du polymère à noyau-coquille comprend au moins 50 à 100% en poids de (méth)acrylates.

**19.** Agent de revêtement, **caractérisé en ce que** l'agent de revêtement comprend un polymère selon au moins l'une quelconque des revendications 15 à 18.

**20.** Procédé pour la préparation d'un revêtement, **caractérisé en ce qu'**un agent de revêtement selon la revendication 19 est appliqué sur un substrat et durci.

**21.** Procédé selon la revendication 20, **caractérisé en ce que** l'agent de revêtement est appliqué sur du bois, du métal, un matériau synthétique, des textiles, des non-tissés et/ou du cuir.

**22.** Procédé selon au moins l'une quelconque des revendications 20 à 21, **caractérisé en ce que** le polymère est transformé avec une diamine et/ou un dihydrazide.

**23.** Procédé selon au moins l'une quelconque des revendications 20 à 22, **caractérisé en ce que** le polymère est soumis à une autoréticulation par un traitement thermique à une température supérieure à 40°C.

**24.** Objet revêtu pouvant être obtenu par un procédé selon au moins l'une quelconque des revendications 20 à 23.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 94025433 A
- DE 2900843
- EP 0005167 A
- US 4191838 A
- WO 2006024538 A
- EP 2005009466 W
- WO 2005000929 A
- US 2004015898 W
- WO 9425433 A
- EP 9401283 W


### In der Beschreibung aufgeführte Nicht-Patentliteratur


- Ullmann's Encyclopedia of Industrial Chemistry
- Chemistry and Technology of Emulsion Polymerisation. Blackwell Publishing, 2005
- **J. O'DONNELL ; E.W. KALER.** *Macromolecular Rapid Communications,* 2007, vol. 28 (14), 1445-1454
- **H. RAUCH-PUNTIGAM ; TH. VÖLKER.** Acryl- und Methacrylverbindungen. Springer, 1967
- Kirk-Othmer, Encyclopedia of Chemical Technology. J. Wiley, 1978, vol. 1, 386ff
- **FOX T. G.** Bull. Am. Physics Soc. 1. 1956, vol. 3, 123
- Polymer Handbook. J. Wiley & Sons, 1975